# EUROPEAN PATENT APPLICATION

(11) **EP 2 127 664 A1**
(43) Date of publication of application: **02.12.2009**
(21) Application number: 08002845.9
(22) Date of filing: 15.02.2008
(51) Int. Cl.: A61K 38/00, C07K 14/705, G01N 33/50

(54) **Change of the load state of MHC molecules by dipeptides**

(71) Applicant: Max-Delbrück-Centrum, 13125 Berlin (DE); Leibnitz-Institut für Molekulare Pharmakologie, 13125 Berlin (DE)
(72) Inventor: Jung, Günther, Prof. Dr., 72076 Tübingen (DE); Rötzschke, Olaf, Dr., 10559 Berlin (DE); Falk, Kirsten, Dr., 10559 Berlin (DE); Kühne, Ronald, 13125 Berlin (DE)
(74) Representative: Graf von Stosch, Andreas

(57) **Abstract**

The present invention relates to methods for changing the load state of MHC molecules, the change in the load state being catalyzed by at least one dipeptide of formula XZ wherein X is an aromatic amino acid and Z is selected from arginine, lysine or histidine. The invention relates further to the use of the dipeptide or to the use of MHC molecules, which load state has been changed, for the treatment of disorders or conditions that are associated with various pathologically excessive or absent immune responses and also for triggering tumor-specific, pathogen-specific or autoreactive immune responses. The invention additionally relates to the use of the dipeptide for the treatment and diagnosis of cancer, infectious diseases, autoimmune diseases and for attenuating aggressive immune reactions, as well as to the preparation of a pharmaceutical composition for the treatment of the mentioned disorders or conditions.

## Description

The present invention relates to methods for changing the load state of MHC molecules, the change in the load state being catalyzed by at least one dipeptide of formula XZ wherein X is an aromatic amino acid and Z is selected from arginine, lysine or histidine. The invention relates further to the use of the dipeptide or to the use of MHC molecules, which load state has been changed, for the treatment of disorders or conditions that are associated with various pathologically excessive or absent immune responses and also for triggering tumor-specific, pathogen-specific or autoreactive immune responses. The invention additionally relates to the use of the dipeptide for the treatment and diagnosis of cancer, infectious diseases, autoimmune diseases and for attenuating aggressive immune reactions, as well as to the preparation of a pharmaceutical composition for the treatment of the mentioned disorders or conditions.

The initiation of the cascade of the immune response proceeds substantially *via* MHC molecules and is based especially on the specific detection and repulsion of pathogenic invaders by the binding of major histocompatibility complex (MHC)-associated peptide antigens to T-cell receptors (TCR). The formation of stable MHC-peptide complexes is of central importance for the triggering of immune responses. Empty MHC-class II molecules are naturally unstable and decompose relatively quickly, while MHC-peptide complexes have a substantially longer half-life. The binding of CLIP or li (class II associated invariant chain peptide (CLIP/li₈₉₋₁₀₂)) immediately after expression of the MHC-class II molecules ensures *inter alia* that the complexes remain stable until they are loaded with antigenic peptides. The dissociation of bound peptides in particular at physiological pH values lasts for a very long time, however, and, depending on the particular peptide in question and on the MHC-class II allele, can last for several hours. Initial investigations found, however, that the low pH values present in late endosomal vesicles drastically accelerate the dissociation of CLIP and accordingly facilitate the association of other peptides (Avva, R.R., and P. Cresswell. 1994, Immunity 1:763-774). Nevertheless, the dissociation rates for many MHC alleles are still so high that the relatively short residence time in endosomal vesicles would not be sufficient to ensure the complete replacement of bonded CLIPs. A physiological cofactor is therefore necessary to catalyze the complete replacement. This is referred to as HLA-DM. HLA-DM is a transmembrane protein that is likewise coded for in the MHC-class II gene locus and accelerates the ligand replacement by binding to the peptide/MHC complex.

The major histocompatibility complex (MHC) gene locus controls a large number of important immunological functions. It is located on the short arm of chromosome 6 of the human genome and codes for several classes of MHC molecules. In humans, the major histocompatibility complex (MHC) is referred to as HLA (human leukocyte antigen) (Wake, C.T. 1986. Molecular biology of the HLA class I and class II genes. Mol Biol Med 3:1-11).

The molecules of class I are referred to as HLA-A, -B and -C. They describe a group of highly polymorphous membrane-located glycoproteins which serve to present endogenously expressed antigens at the cell surface. They are expressed in almost all cells of the human body and form heterodimers from the heavy MHC class I chains (HLA-A, -B or -C) and a small protein not coded for in the MHC gene locus, ββ2 microglobulin (β2m). The heavy α-chains possess a short cytoplasmic domain, a transmembrane domain and three extracellular domains that are linked non-covalently with β2m. The antigens bound by MHC class I are peptides having almost exculsively a length of from 9 to 11 amino acids. The peptide binding site is formed solely by the α-chain and consists of a β-sheet consisting of eight strands, on which the peptides are clamped between two α-helices and fixed at both ends by hydrogen bridge bonds between the N- or C-termini of the bound peptide and the MHC complex.

The molecules of class II likewise describe a group of highly polymorphous membrane-located glycoproteins which have the function of antigen presentation. Unlike MHC class I molecules, however, these are normally expressed only in a group of special antigen-presenting cells (APC) and serve predominantly for the presentation of peptides of exogenously absorbed proteins. In humans, a distinction is made between three different types of class II molecules: HLA-DP, -DQ and -DR, of which a large number of different alleles in turn exist. The αβ-heterodimers formed consist of an α- and a β-chain, both of which are coded for in the MHC. Both chains possess a short cytoplasmic domain, a transmembrane domain and two extracellular domains (α1 and α2 or β1 and β2). The peptide binding groove is formed by the α1 and β1 domains of both chains (Brown, J.H., T.S. Jardetzky, J.C. Gorga, L.J. Stern, R.G. Urban, J.L. Strominger, and D.C. Wiley. 1993. Three-dimensional structure of the human class II histocompatibility antigen HLA-DR1. Nature 364:33-39). As with class I molecules, in this case the peptide is bound on a β-sheet between a plurality of α-helical structures. Unlike MHC class I molecules, however, the ends of the peptide binding site are open, so that the peptides are able to protrude from the complex (Rajnavolgyi, E., A. Horvath, P. Gogolak, G.K. Toth, G. Fazekas, M. Fridkin, and I. Pecht. 1997. Characterizing immunodominant and protective influenza hemagglutinin epitopes by functional activity and relative binding to major histocompatibility complex class II sites. Eur J Immunol 27:3105-3114). *In vivo,* the size of the bound peptides is normally in a range from 13 to 25 amino acids (AA) (Chicz, R.M., R.G. Urban, W.S. Lane, J.C. Gorga, L.J. Stern, D.A. Vignali, and J.L. Strominger. 1992. Predominant naturally processed peptides bound to HLA-DR1 are derived from MHC-related molecules and are heterogeneous in size. Nature 358:764-768, Rudensky, A., P. Preston-Hurlburt, S.C. Hong, A. Barlow, and C.A. Janeway, Jr. 1991. Sequence analysis of peptides bound to MHC class II molecules. Nature 353:622-627), but only 13 of them are fixed in the peptide binding fold of the MHC molecule. Crystallographic studies revealed that these 13 AAs are bound in an elongated conformation, similar to a polyproline type II helix. The AAs projecting from the peptide binding groove, on the other hand, can also assume other conformations (Jardetzky, T.S., J.H. Brown, J.C. Gorga, L.J. Stern, R.G. Urban, J.L. Strominger, and D.C. Wiley. 1996. Crystallographic analysis of endogenous peptides associated with HLA-DR1 suggests a common, polyproline II-like conformation for bound peptides. Proc Natl Acad Sci U S A 93:734-738). More detailed studies of the peptide bond showed that 9 AAs of the peptide are responsible for the affinity of the bond (Hill, J.A., S. Southwood, A. Sette, A.M. Jevnikar, D.A. Bell, and E. Cairns. 2003. Cutting Edge: The Conversion of Arginine to Citrulline Allows for a High-Affinity Peptide Interaction with the Rheumatoid Arthritis-Associated HLA-DRB1*0401 MHC Class II Molecule. J Immunol 171:538-541). Lengthening of the peptides in either direction does not increase the affinity of the bond (Siklodi, B., A.B. Vogt, H. Kropshofer, F. Falcioni, M. Molina, D.R. Bolin, R. Campbell, G.J. Hammerling, and Z.A. Nagy. 1998. Binding affinity independent contribution of peptide length to the stability of peptide-HLA-DR complexes in live antigen presenting cells. Hum Immunol 59:463-471).

Experiments with various alleles of the MHC class II molecule HLA-DR identified over the course of the binding fold a plurality of "binding pockets", which are critical for the affinity of the peptide for the particular allele in question (O'Sullivan, D., T. Arrhenius, J. Sidney, M.F. Del Guercio, M. Albertson, M. Wall, C. Oseroff, S. Southwood, S.M. Colon, F.C. Gaeta, et al. 1991. On the interaction of promiscuous antigenic peptides with different DR alleles. Identification of common structural motifs. J Immunol 147:2663-2669; and Sette, A., J. Sidney, C. Oseroff, M.F. del Guercio, S. Southwood, T. Arrhenius, M.F. Powell, S.M. Colon, F.C. Gaeta, and H.M. Grey. 1993. HLA DR4w4-binding motifs illustrate the biochemical basis of degeneracy and specificity in peptide-DR interactions. J Immunol 151:3163-3170). For all allelic forms of HLA-DR, the binding in the first pocket close to the N-terminal end of the peptide appears to be decisive for the stability of the complex as a whole (Hammer, J., P. Valsasnini, K. Tolba, D. Bolin, J. Higelin, B. Takacs, and F. Sinigaglia. 1993. Promiscuous and allele-specific anchors in HLA-DR-binding peptides. Cell 74:197-203). However, the pockets at the relative positions P3, P4, P6, P7 and P9 as well as pockets at other positions of the binding fold appear, in an allele-specific manner, to be very important for the affinity of bound peptides (Southwood, S., J. Sidney, A. Kondo, M.F. del Guercio, E. Appella, S. Hoffman, R.T. Kubo, R.W. Chesnut, H.M. Grey, and A. Sette. 1998. Several common HLA-DR types share largely overlapping peptide binding repertoires. J Immunol 160:3363-3373). However, all alleles, even in the most stringent position, permit the binding of various amino acid side chains of the bound peptide, so that a large number of very different peptides can be bound to the same HLA allele (McFarland, B.J., and C. Beeson. 2002. Binding interactions between peptides and proteins of the class II major histocompatibility complex. Med Res Rev 22:168-203). The stability of the bond is determined, as well as by these allele-specific binding pockets, also by a number of hydrogen bridge bonds from highly conserved parts of the MHC molecule to the peptide backbone of the antigen. Unlike in MHC class I complexes, these are distributed over the entire peptide binding groove (Batalia, M.A., and E.J. Collins. 1997. Peptide binding by class I and class II MHC molecules. Biopolymers 43:281-302).

Strength and direction of the immune response is largely controlled by CD4+ T cells. As 'helper' T cells they drive proinflammatory responses by activating antibody-producing B cells and 'cytotoxic' CD8+ T cells, and as suppressor cells they contain over-shooting immune reactions by neutralizing other immune cells. The importance of these cells is particularly evident in autoimmune diseases. The strongest genetic link is here usually the class II MHC molecule, the polymorphic peptide receptor encoded by the 'major histocompatibility gene complex' (MHC) which presents the antigen to the CD4+ T cell.

Class II MHC molecules are loaded inside the cell in dedicated endosomal compartments (MCII). The MCII compartment is generated after fusion with acidic lysosomal-like vesicles containing proteins internalized by endocytosis or receptor-mediated uptake. MHC-loading is facilitated by HLA-DM, a chaperone stabilizing the 'peptide receptive' state. While this conformation has not yet been structurally defined, is characterized kinetically by rapid on- and off-rates of the peptide ligands.

Transitions between the receptive and non-receptive form of class II MHC molecules are evident also on the cell surface. In the absence of an active chaperone, however, the equilibrium is largely shifted towards the non-receptive conformation. As a consequence MHC molecules, once they have lost their ligand, rapidly convert into the stable inactive state. As a safeguard mechanism it prevents 'accidental' loading of class II MHC molecules with free peptides from the extracellular space and by that insures that the ligands presented on the cell surface accurately reflect the peptide composition inside the MCII compartment.

Starting from prior knowledge, changing the load state of MHC molecules with antigens appears to be a very promising approach to the therapy of pathological immune responses as well as of various pathologically excessive or absent immune responses. However, very different *in vitro* approaches for accelerating these processes have not hitherto yielded any practicable loading rates for MHC molecules or produced any findings which indicate corresponding usability of such MHC molecules.

For example, Jensen *et al.* (Jensen et al., J. Exp. Med., 1990, 171 :1779-84) describe increasing the loading of MHC class II molecules by lowering the pH value. Jensen *et al.* (1990, supra) put forward the hypothesis that peptide replacement reactions might occur at the cell surface of activated APCs. Within the context of an infection, the density of presented, normally edited self-peptides, which otherwise are presented below the limiting value for activation of autoreactive T-cells, might be increased thereby. Jensen *et al.* (1990, supra) put forward local lowering of the pH value as a possible cause of such extracellular replacement reactions. However, local lowering of the pH value is possible substantially only in the case of MHC molecules that are soluble at such a pH, but not in the case of MHC molecules that are already present in insoluble form at such a pH. Furthermore, the loading of MHC molecules with antigens by lowering the pH value can be used only very briefly, or only after fixing of the cells, owing to the cell-damaging, non-physiological action. It is therefore not possible to use the process described according to Jensen *et al.* (1990, supra) for *in vivo* situations.

A further approach to the loading of MHC molecules with antigens takes place *via* the use of the natural catalyst for MHC ligand replacement, HLA-DM (Weber et al., J. Immunol., 2001, 167:5167-74). The method described by Weber *et al.* permits very efficient loading in principle, but the preparation of the HLA protein is very complex and expensive and accordingly is not obtainable commercially. Furthermore, the loading of MHC molecules with antigens in this method requires the presence of detergents and can only be carried out efficiently at a low pH value. Because the use of a low pH value and the use of detergents in Weber *et al.* (2001, supra) lead to non-physiological conditions, an *in vivo* use of the loaded MHC molecules obtained by this method is excluded.

A further alternative for the loading of MHC molecules with antigens is the use of destabilising detergents, which presumably are able to accelerate the loading of MHC class II molecules on account of their complex-destabilising influence (see Roof et al., Proc. Natl. Acad. Sci. U.S.A., 1990, 87:1735-9; Avva and Cresswell, Immunity, 1994, 1:763-74). However, destabilising detergents exhibit only a comparatively low activity in the loading of MHC molecules and have the obvious disadvantage that, after addition, it is not possible, or is possible only with extreme difficulty, to separate them from the MHC molecules. The separation of destabilising detergents and loaded MHC molecules therefore appears to be impossible under *in vivo* conditions, and MHC molecules loaded with the aid of destabilising detergents could therefore be introduced *in vivo* only together with the destabilising detergents used. However, because the use of such detergents leads to the decomposition or partial decomposition of cells, *in vivo* use of the loaded MHC molecules obtained by the method described by Roof et al. (Proc. Natl. Acad. Sci. U.S.A., 1990, 87:1735-9) or Avva and Cresswell (Immunity, 1994, 1:763-74) is not conceivable.

The best results obtained hitherto when loading MHC molecules with antigens were achieved by the use of low molecular weight compounds, such as so-called small molecules such as, for example, para-chlorophenol, which act as H donors (see Falk et al., J. Biol. Chem., 2002, 277:2709-15; and Marin-Esteban et a/., J. Autoimmun., 2003, 20:63-9; and WO 03/016512). In these studies it has been shown that so-called small molecules with H-donor properties possess the ability to catalyse the loading of MHC class I and class II molecules with peptides at physiological pH value or to replace bound antigens at the surface of APCs at physiological pH value. However, all the substances identified in these studies that are capable of influencing MHC class II ligand interactions can be used only at relatively high, non-physiological concentrations. For example, the use of phenol requires a concentration of 30 mM, n-propanol even a concentration of over 200 mM, which concentrations already have a considerable toxic action *in vivo.* In order to develop their activity, concentrations that are toxic *in vivo* would have to be used, so that it is neither possible nor conceivable to test their activity in animal models and accordingly their potential association with various pathologically excessive or absent immune responses.

A still further approach to change the load state of MHC molecules was the use of adamatyl compounds as described in WO2006/029891. Adamantyl compounds, however, are generally non-natural compounds which may exhibit toxicity. Even if the use of adamantyl compounds *in vitro* may not cause serious concerns, these compounds may still have a toxic effect if administered *in vivo,* e.g. as a pharmaceutical composition.

Although different approaches have been used to solve the problem of catalysis for an efficient changing of the load state of MHC molecules, there is a need in the art for alternative solutions for that problem.

The object underlying the present invention is, therefore, to provide a method for changing the load state of MHC molecules, which method permits an efficient unloading or loading of the MHC molecules with ligands, the replacement of ligands on the surface of MHC molecules, the decrease or removal of ligands on the surface of MHC molecules, and the subsequent *in vivo* use thereof.

A further object of the present invention is to provide therapeutic agents or diagnostic agents, or methods which can be carried out by such therapeutic agents or diagnostic agents, for the treatment or detection of disorders or conditions that are associated with various pathologically excessive or absent immune responses.

According to the invention, the object underlying the invention is achieved by a method for changing the load state of MHC molecules. This method comprises the following steps:
a) providing a composition comprising MHC molecules;
b) changing the load state of the MHC molecules by adding at least one dipeptide having the following formula:
   XZ
   wherein X is an aromatic amino acid and Z is selected from arginine, lysine or histidine, and wherein the dipeptide is modified or unmodified; and
c) isolating the MHC molecules.

Alternatively, this method comprises the following steps:
a) providing a composition comprising MHC molecules;
b') changing the load state of the MHC molecules by adding at least one dipeptide having the following formula:
   XZ
   wherein X is an aromatic amino acid and Z is selected from arginine, lysine or histidine, and wherein the dipeptide is modified or unmodified, and by adding at least one ligand of MHC molecules; and
c) isolating the MHC molecules.

To induce antigen-specific T-cell responses e.g. in the context of vaccinations or immune therapies, it is necessary to change the load state of MHC molecules, in particular for loading with e.g. antigenic substances. The process is hindered, however, since most MHC molecules are already loaded with e.g. an antigenic peptide or - due to a safeguard mechanism - have converted into a non-receptive state. Thus, the loading of antigens is inefficient and requires therefore elevated amounts of e.g. antigenic peptides for loading the MHC molecules with antigens to induce a protective immune response.

Thus, the method according to the invention uses at least one dipeptide of formula XZ for changing the load state of MHC molecules. Changing the load state may either mean unloading loaded MHC molecules (according to a first method of the present invention) or, in the presence of at least one ligand of MHC molecules, loading of unloaded MHC molecules or reloading already loaded MHC molecules with the new ligand(s) (according to a second method of the invention).

That dipeptide is advantageous over the prior art, in particular adamantyl compounds, since its peptide backbone allows localization of the dipeptide precisely over the P1 pocket so that it guides the catalytic group (e.g. amino acid side chain or modified residue) into the P1 pocket. The interaction occurs via H-bond and is well characterized. Thus, development of specific dipeptides can be achieved by rational design for any specific MHC molecule. Moreover, as natural or natural-like compound it exhibits significantly lower toxicity if used *in vivo.*

MHC molecules used in a method according to the present invention can be obtained from various natural sources or by means of recombinant methods. Natural sources of MHC molecules within the scope of the present invention are, for example, cells or tissues of human or animal origin, more preferably endogenous or non-endogenous dendritic cells, such as, for example, maturated and non-maturated dendritic cells, as well as B-cells or macrophages or other antigen-presenting cells. Natural sources of MHC molecules within the scope of the present invention likewise include preferably body fluids containing the above-defined cells, for example blood, lymph or tissue, etc.

MHC molecules obtained from natural sources and used in a method according to the present invention can be employed in isolated form or together with the cells with which they are associated.

On the one hand, MHC molecules can be obtained from natural sources, i.e. from cells, body fluid, lymph, etc., and isolated by biochemical purification methods, for example chromatographic methods, centrifugation methods, dialysis methods, antibody binding methods, etc. This is carried out, for example, by cleavage of the extracellular portion of the cells contained therein by washing steps and/or cleavage with proteases from the cells contained, for example, in body fluids or lymph and associated with MHC molecules. The MHC molecules are then preferably isolated from the cleaved extracellular fraction by biochemical purification methods, for example by antibody binding methods. The isolated MHC molecules so obtained can then be used in a method according to the invention.

Alternatively, MHC molecules from natural sources can be used in a method according to the invention together with their associated cells, without further isolation of the MHC molecules. To this end, the cells are typically isolated as such together with the MHC molecules. Methods of obtaining cells are known to a person skilled in the art. For example, in order to obtain MHC molecules within the scope of the present invention from natural sources, there are taken from a patient preferably dendritic cells, particularly preferably maturated and/or non-maturated dendritic cells, or B-cells or macrophages, or other cells expressing MHC molecules, individually or in collections of a plurality of cells, and are purified. Purification methods for cells are likewise known from the art to a person skilled in the art. For example, for the purification of cells, the constituents of a cell or tissue sample from a natural source are subjected to separation on the basis of their density. Separation on the basis of density can be carried out by means of chromatographic methods, for example by size-exclusion chromatography or FPLC, or by means of centrifugation, preferably density gradient centrifugation, for example over a Ficoll separating solution. After separation of the constituents on the basis of their density, the cells are typically concentrated by adherence of the desired target cells to a matrix or a solid phase, for example to nylon wool, and undesired cells are depleted. A solid phase or a matrix within the scope of the present method is any surface to which MHC molecules can be bound directly, *via* a linker, labeling or *via* their affinity. Alternatively, the cells can be concentrated by chromatographic methods, for example by chromatographic size-exclusion methods or FPLC methods. If necessary, the cell count present in the cell suspension can be determined after a first concentration operation. The cells are then typically separated from the resulting cell suspension by chromatographic methods, by binding to a solid phase, by magnetic sorting by means of a MACS method (Magnetic Activated Cell Sort) or by comparable methods. Magnetic cell sorting permits, for example, the quantitative yield of highly pure cell populations from different tissues. The cells are preferably negatively sorted during separation, i.e. all the cells that are not desired are depleted from the cell mixture. Alternatively, the desired cells can likewise be sorted preferably positively directly from the cell mixture. In both cases, the cells can be labelled preferably with a monoclonal antibody specific for the cell type, which antibody can be bound to a solid phase or to particles, for example to superferromagnetic particles (microbeads). Alternatively, a first specific monoclonal antibody can be recognized by a second antibody which is bound to a solid phase, conventionally microbeads, and recognizes the Fc fraction of the first antibody. After a chromatographic method or binding to a solid phase, the cells can be separated from the column or the solid phase, typically by elution. After magnetic sorting by means of a MACS method and binding to microbeads, the cells can typically be separated in a magnetic field over a column. Details relating to the method are evident from the information given by the manufacturer of the microbeads that are used (e.g. Miltenyi Biotech, Bergisch Gladbach, Germany). Purification of the cells is typically carried out at any time, while cooling, preferably while cooling with ice. If required, the cell suspensions obtained are washed at any time during the purification, once or several times, with suitable buffer solutions, for example PBS. When purification is complete, the resulting cells are either stored or used directly. The cells are conventionally stored in a buffer which maintains the cells under physiological conditions and does not cause osmotic pressure. Suitable buffers are, for example, PBS buffer, BSA-PBS buffer (PBS buffer with bovine serum albumin (BSA)), FCS-PBS buffer (PBS buffer with fetal calf serum (FCS)), phosphate buffer, TRIS-HCl buffer, tris-phosphate buffer, or further suitable buffers, such as Dulbecco's modified Eagle Medium (DMEM) or RPMI with 5-10% FCS, the two last-mentioned buffers being suitable in particular for the storage of cells. The cells can optionally be stored and/or used in nutrient medium, for example FCS/10% DMSO.

MHC molecules for use in one of the methods according to the invention can also be made available by recombinant methods. Methods for the recombinant preparation of MHC molecules are known to a person skilled in the art (see, for example, J. Sambrook; E.F. Fritsch; T. Maniatis, 2001, "Molecular cloning : a laboratory manual", Cold Spring Harbor, NY : Cold Spring Harbor Laboratory Press). To this end, vectors that code for MHC molecules are typically introduced into suitable cell expression systems that express MHC molecules and are then harvested, and the MHC molecules are purified by means of biochemical purification methods, for example chromatographic methods, centrifugation methods, dialysis methods, etc.

MHC molecules used in a method according to the invention are typically MHC monomers or multimers, preferably MHC monomers or multimers of classes I and II. MHC molecules obtained by means of a recombinant method are typically in monomeric form. MHC molecules obtained from a natural source are likewise conventionally in monomeric form. In order to convert such monomeric MHC molecules into a multimeric form, MHC molecules isolated from natural sources or prepared by recombinant methods can be biotinylated and then bound. Binding of the biotinylated MHC molecules to fluorescent-labelled streptavidin molecules yields multimeric MHC complexes, preferably tetrameric complexes, because there are 4 biotin binding sites on streptavidin. Alternatively, MHC molecules isolated from natural sources or prepared by recombinant methods can be multimerised, preferably to give MHC tetramers or pentamers of classes I and II. To this end, a self-arranging coiled-coil domain is preferably co-expressed together with a recombinant MHC molecule. Alternatively to the multimerisation methods described here, any further known methods for the multimerisation of MHC molecules can be used. Such methods are known to a person skilled in the art. A further multimerisation possibility consists in the covalent or non-covalent binding of the molecules to surfaces, such as, for example, culture plates or so-called microbeads. Both are already used for stimulating T-cells, it being possible to use microbeads, in the case where they contain an iron core (e.g. Dynabeads, Dynal Biotech GMBH), also for the selective purification of the cells by means of magnets. For the latter purposes, it is also possible to use monomeric MHC molecules coupled to so-called nano-beads (e.g. Miltenyi Biotec). Multimeric MHC molecules can likewise be obtained commercially. For example, tetramers can be obtained from Becton Dickinson, Beckman Coulter, and pentamers can be obtained from Proimmune. MHC multimers loaded with ligands by a method according to the present invention are preferably capable of binding selectively to T-cells whose T-cell receptor specifically recognises the ligand loaded on the complex.

MHC molecules used in a method according to the invention can be labelled. The MHC molecules whose load state is changed according to a method of the present invention, preferably MHC multimers, are preferably labelled before their load state is changed. The MHC molecules preferably contain labels permitting the generation of a signal that can be detected directly or indirectly. The following modifications are known to the person skilled in the art:
(i) radioactive modifications, e.g. radioactive phosphorylation or radioactive labelling with sulfur, hydrogen, carbon, nitrogen,
(ii) colored groups (e.g. digoxygenin, etc.),
(iii) fluorescent groups (e.g. fluorescein, etc.),
(iv) chemoluminescent groups,
(v) groups for immobilization on a solid phase (e.g. biotin, Strep tag, antibodies, antigens, etc.),
or combinations of modifications according to two or more of the modifications mentioned under (i) to (v).

MHC molecules used in a method according to the invention typically occur in a closed, unloaded, non-receptive conformation or in an open, receptive conformation. In the closed, non-receptive conformation, the MHC molecules are preferably not capable of taking up or giving up ligands. A transition between these different conformations of an MHC molecule typically takes place by a corresponding shift of the equilibrium of the reaction, i.e. a shift of equilibrium between the closed, unloaded, non-receptive conformation; the open, unloaded, receptive conformation; and the open, loaded, receptive conformation. In the methods according to the invention, the MHC molecules are converted by the dipeptide that are used preferably from a closed, unloaded, non-receptive conformation to an open, unloaded, receptive conformation. Alternatively, the MHC molecules are converted directly from a closed, unloaded, non-receptive conformation to an open, loaded, receptive conformation. If the MHC molecule is converted into an open, receptive state as a result of the catalytic action of the dipeptide, or if it is in an open, receptive state, the MHC molecules can be loaded with ligands or a replacement of ligands can take place on the MHC molecule. In other words, the MHC molecule in the open, unloaded, receptive conformation is preferably capable of taking up a ligand. Alternatively, the ligand of an MHC molecule in the open, loaded, receptive conformation can be replaced. In a further alternative, it is also possible to carry out loading of the MHC molecule with ligands directly from the closed, unloaded, non-receptive conformation using the dipeptide. Moreover, using the dipeptide, it is possible to unload a loaded MHC molecule, preferably from the open, loaded, receptive conformation.

Monomeric or multimeric MHC molecules used in a method according to the invention can be provided in an unloaded form or in a form loaded with ligands. In the case of preparation by recombinant methods, the MHC molecules are typically unloaded after their purification. It is, however, likewise possible for MHC molecules isolated from natural sources or the MHC molecules isolated together with their associated cells to be unloaded after their purification, e.g. by a method of the present invention. Such unloaded MHC molecules can then be loaded with desired ligands by a method according to the present invention. A change in the load state of MHC molecules, in that case, preferably means loading of unloaded MHC molecules with ligands. Alternatively, MHC molecules may be provided in a form loaded with ligands both after their preparation by recombinant methods or after their isolation from natural sources. The number of ligands on the surface of such loaded MHC molecules can be decreased or the ligands can be removed completely in a method according to the invention. A change in the load state of MHC molecules in this case therefore preferably means decreasing the load state of (previously loaded) MHC molecules with ligands, and optionally the complete removal of ligands from (previously loaded) MHC molecules. In a further alternative, ligands of (previously loaded) MHC molecules can be replaced (substituted) by another desired class of ligands by means of the methods according to the invention. A change in the load state of MHC molecules in this case therefore preferably means replacing ligands of (previously loaded) MHC molecules by desired ligands. To this end, ligands already present on the MHC molecule are typically removed or decreased beforehand by a method according to the invention (step (i)), and then MHC molecules are loaded again with desired ligands by a method according to the invention (step (ii)). According to the invention, steps (i) and (ii) can also be carried out simultaneously.

In one preferred embodiment according to the invention, steps (i) and (ii) are carried out simultaneously and the dipeptide XZ and the ligand of MHC molecules of step b') are linked together. The linkage can be either covalent or non-covalent, permanent or transient. Preferably, the linkage is covalent and permanent. Further, the linkage can be either direct or indirect. Using a direct linkage, the one or more ligands are directly attached to the N-terminus, C-terminus and/or the side chain of the dipeptide. An indirect linkage, instead, requires a linker which can be an amino acid, peptide, non-peptide or chemical linker. It is to be understood in the context of the invention that the dipeptide may or may not be modified by one or more compounds or groups as described below. Non-covalent linkages can be realized by e.g. complexing the dipeptide by the ligand. The ratio of both compounds can thereby vary depending on the dipeptide and/or ligand used.

In a preferred embodiment of the method according to the invention, ligands of MHC molecules are native and non-native compounds which bind to MHC molecules under physiological conditions. In this connection, particularly preferred ligands of MHC molecules are antigens, in particular tumor- or pathogen-specific antigens, peptide antigens, tissue-specific self-antigens, antigens of autoreactive T-cells or fragments of such antigens, preferably having a length of from 8 to 25 amino acids, more preferably having a length of from 8 to 15 amino acids. Likewise preferably, ligands of MHC molecules are larger peptide fragments, protein domains, complete proteins, protein mixtures, complex protein mixtures and/or cell lysates, preferably tumor cell lysates.

In a particular embodiment of the subject-matter according to the invention, the composition provided in step (a) in a method according to the invention can be an aqueous composition. The composition preferably comprises a buffer in addition to the MHC molecules. For example, the following buffers can be used: PBS buffer (for example pH 5.0-8.5, preferably pH 7.0-8.0, 50-200 mM NaCl, preferably about 137.0 mM NaCl, 0.5-5 mM KCI, preferably 2.7 mM KCI, preferably 1-10 mM Na₂HPO₄, preferably 4.3 mM Na₂HPO₄, 0.1-5 mM KHPO₄, preferably 1.4 mM KHPO₄), BSA-PBS buffer (for example pH 5.0-8.5, 1-15 wt.% bovine serum albumin (BSA), preferably pH 5.0-8.0, 50-200 mM NaCl, preferably about 137.0 mM NaCl, 0.5-5 mM KCI, preferably 2.7 mM KCI, preferably 1-10 mM Na₂HPO₄, preferably 4.3 mM Na₂HPO₄, 0.1-5 mM KHPO₄, preferably 1.4 mM KHPO₄), FCS-PBS buffer (for example pH 5.0-8.5, 1-15 wt.% fetal calf serum (FCS), preferably pH 5.0-8.0, 50-200 mM NaCl, preferably about 137.0 mM NaCl, 0.5-5 mM KCI, preferably 2.7 mM KCI, preferably 1-10 mM Na₂HPO₄, preferably 4.3 mM Na₂HPO₄, 0.1-5 mM KHPO₄, preferably 1.4 mM KHPO₄), phosphate buffer (for example pH 5.0-8.5, preferably pH 7.0, 20-80 mM Na₂HPO₄, preferably 57.7 mM Na₂HPO₄, 10-60 mM NaH₂PO₄, preferably 42.3 mM NaH₂PO₄), TRIS-HCl buffer (for example 0.5-3.0 M TRIS with HCl(conc.), preferably 1-1.5 M TRIS adjusted to pH 6.8-8.0 with HCl(conc.)), tris-phosphate buffer (for example 20-80 mM Na₂HPO₄, 10-60 mM NaH₂PO₄, 0.5-3.0 M TRIS adjusted to pH 6.0-8.5 with HCl(conc.)), or further suitable buffers. The mentioned concentrations are preferably the concentrations in the buffer solutions before they are added to the composition. From 0 to 15.0 vol.% are typically added to the composition. The composition provided in the method according to the invention can likewise comprise organic solvents. Organic solvents are preferably selected from the following group: DMSO, ethanol, methanol, acetone and the like, particularly preferably from 0.1 to 1% DMSO. The pH of the composition is typically from 6.0 to 8.5, preferably from 6.5 to 7.5. In addition to the aqueous solution or the organic solvent, the composition can optionally comprise a polar solvent, for example dimethyl sulfoxide (DMSO), dimethylformamide (DMF), TFE (tetrafluoroethylene), hexamethylphosphoric acid triamide (HMPT), hexamethyl-phosphoramide (HMPA) or the like. The final concentration of the polar solvent in the composition can preferably be from 0.05 to 15 wt.%, more preferably from 0.1 to 5 wt.% and yet more preferably from 0.5 to 2 wt.%. Likewise preferably, the loading is carried out in DMEM or RPMI buffers.

In step (b) or (b') of the method according to the invention, there is typically added to the provided composition from step (a) at least one dipeptide to a final concentration of from 0.001 to 500 mM, preferably in a concentration of from 0.001 to 250 mM, more preferably in a concentration of from 0.001 to 100 mM.

The molar ratio of MHC molecule to ligand, if added, is typically from 0.1:10 to 10:0.1. The molar ratio of ligand to MHC molecule is preferably from 0.5:5 to 5:0.5, particularly preferably from 0.75:2.5 to 2.5:0.75. In a particular embodiment of the method according to the invention, changing the load state of MHC molecules is preferably carried out at a temperature of from 20 to 40°C, more preferably at a temperature of from 24 to 39°C and most preferably at a temperature of from 36 to 38°C.

When the load state of MHC molecules is changed with ligands in step (b') by a method according to the invention, the amount of ligands loaded onto or replaced on an MHC molecule by the method according to the invention is preferably controlled. For example, it is possible by means of the method according to the invention, preferably *in vitro,* to achieve a load state that leads to an equal load, to an increase or alternatively to a decrease in the loading of MHC molecules with ligands as compared with the loading of MHC molecules under *in vivo* conditions, or as compared with the loading of the MHC molecules contained in the composition before the method according to the invention was carried out. In a particularly preferred embodiment of the method according to the invention, changing the load state of MHC molecules can lead to the new loading of previously unloaded MHC molecules, to removal of some or all of the ligands that were already present, or to replacement of ligands that were already present by different, desired ligands. Methods for determining load states under *in vivo* and *in vitro* conditions are known to a person skilled in the art.

In a preferred embodiment of the method according to the invention, a change in the load state of unloaded MHC molecules with ligands is achieved in step (b') by addition of potential ligands, in particular peptides, for example peptide ligands, to the composition comprising MHC molecules according to method step (a) and the dipeptide according to method step (b'). The concentrations of MHC molecules, ligands and the dipeptide are preferably as described above. Method steps (a), (b') and (c) of the method according to the invention described here for changing the load state of MHC molecules can be carried out in any desired sequence or in parallel. For example, steps (a) and (b') can take place in parallel. Also, instead of providing the composition comprising MHC molecules, the dipeptide, preferably in solution, can first be provided in a method step and then the other method steps, such as, for example, addition of ligands and/or MHC molecules, can be carried out. Likewise, all the steps (a), (b') and (c) can be carried out simultaneously.

In another alternative embodiment of the method according to the invention, a change in the load state of MHC molecules already loaded with ligands is achieved in a step (b') by replacing some or all of the ligands already present on the MHC molecule by another desired class of ligands. To this end, in a first step (i), a decrease in the load density of ligands on the MHC molecules to a desired load state is preferably achieved as described in greater detail hereinbelow. To this end, some or optionally all of the ligands already bound to the MHC molecule are typically removed e.g. by a washing step using the dipeptide. The MHC molecules can thereby be immobilized on a solid phase, for example Sepharose, beads, microbeads, etc., or *via* linkers obtainable in the art, for example tags such as a His-tag. Any suitable buffer described above can be used in the washing step. Thereafter, in a second step (ii), the desired ligand and optionally a further amount of the dipeptide are typically added to the composition and incubated together with the MHC molecules. The new ligand thereby binds to the MHC molecule, preferably by a shift of the concentration equilibrium, i.e. the new ligand is present preferably in such a concentration that displacement of the previously bound ligand by the new ligand takes place using the dipeptide. Method steps (a), (b') and (c) described here of the method according to the invention can therefore be carried out in any desired sequence or in parallel. For example, steps (a), (b') and (c) can taken place in parallel. Also, instead of providing the composition comprising MHC molecules, the dipeptide, preferably in solution, can first be provided in a first method step and then the MHC molecule can be added thereto.

In an alternative embodiment of the method according to the invention, a change in the load state of MHC molecules already loaded with ligands leads to a decrease in the load density of ligands on the MHC molecules or to unloading of MHC molecules. To this end, some or optionally all of the ligands already bound to the MHC molecule are typically removed using the dipeptide. For this purpose, after providing the composition comprising MHC molecules according to method step (a) and addition of the dipeptide according to method step (b), a washing step is preferably carried out, which step separates from the MHC molecules the ligands detached from the MHC molecule by means of the dipeptide. To this end, the MHC molecules can be immobilized on a solid phase, for example Sepharose, beads, microbeads, for example *via* linkers obtainable in the art, for example tags such as a His-tag, etc. Any suitable buffers described above can be used in the washing step. Method steps (a), (b) and (c) of the embodiment described here of the method according to the invention can be carried out in any desired sequence or in parallel. For example, steps (b) and (c) can take place in parallel. Also, instead of providing the composition comprising MHC molecules, the dipeptide, preferably in solution, can first be provided in a method step and then the other steps, such as the addition MHC molecules, can be carried out. Likewise, all the steps (a), (b) and (c) can be carried out simultaneously.

In a particular embodiment of the method according to the invention, the change in the load state of MHC molecules is typically carried out on an MHC molecule of class I or II, at a peptide binding region of the MHC molecule. The peptide binding region is preferably a peptide binding region of an MHC class I or MHC class II molecule. Typically, the peptide binding region of an MHC I molecule is formed by the α-chain of the MHC class I molecule and consists of a β-sheet formed of eight strands, on which the peptides are clamped between two α-helices and are fixed at both ends by hydrogen bridge bonds between the Nor C-termini and the MHC class I molecule. The peptide binding region of the MHC class I molecule conventionally contains a plurality of binding pockets. The specificity of a ligand at the peptide binding region of an MHC class I molecule is typically produced by binding the amino acid side chains with in each case one of these binding pockets. In the case of the MHC I molecule, the binding pocket is preferably selected from the binding pockets A, B, C, D, E and F, which preferably take up the side chains of the ligands. The peptide binding region of an MHC II molecule is typically formed by the α₁ and β₁ domains of the α- and β- chain forming the MHC II molecules. The peptide binding region of the MHC II molecule likewise conventionally contains one or more binding pockets. In the case of the MHC II molecule, the binding pocket is preferably selected from the binding pockets P1, P3, P4, P6, P7 and P9, which preferably take up the side chains of the ligands. Likewise preferably, the specificity of the ligand at the peptide binding region, or preferably at a peptide binding pocket, is produced by binding of the amino acid side chains with the described binding pocket. Binding of the ligand is conventionally produced in particular by binding to binding pocket P1 of an MHC molecule of class II, which in the case of HLA-DR molecules differs substantially only by the occupancy of the dimorphic residue β86. Binding of the ligands, or the stability of the complexes, can additionally be influenced by further regions which lie outside the actual peptide binding region. For example, pH-dependent destabilization is influenced considerably by a region which is located beneath the binding groove. At this location there is a histidine residue (His33), which bridges the α₁ domain with the β₁ domain and is influenced directly by protons and possibly also other H donors (Rötzschke et al. PNAS, 2002, 99: 16946-16950).

In a step (c) of the method according to the invention, isolation of the MHC molecules loaded with ligands is carried out, for example by a biochemical or biophysical isolation method or by a method of isolating cells, as described hereinbefore.

Biophysical and biochemical detection methods or isolation methods within the scope of the present invention are preferably spectroscopic methods, sequencing reactions, immunological methods or chromatographic methods. Within the scope of the present invention, spectroscopic methods are preferably selected from a NMR, light scattering, Raman, UV, VIS, IR, circular dichroism method, analysis by mass spectrometry (MS) or X-ray structure analysis. A sequencing reaction is understood as meaning preferably peptide or nucleic acid sequencing. Immunological methods are preferably selected from ELISA methods, antibody binding assays, immunofluorescence detection methods or Western blots. Chromatographic methods according to the present invention are preferably adsorption chromatography, distribution chromatography, ion-exchange chromatography, (size-)exclusion chromatography or affinity chromatography. There is further carried out preferably chromatography by means of Ni-NTA agarose, HPLC, FPLC, or reversed-phase (RP) chromatography.

According to step (b) or (b') of the method of the present invention, at least one dipeptide of formula XZ is added to the MHC molecules for changing the load state of MHC molecules. Advantageously, that dipeptide is localized precisely over the P1 pocket so that it guides the catalytic group (e.g. amino acid side chain or modified residue) into the P1 pocket.

In a preferred embodiment of the present invention, the aromatic amino acid X is selected from the group consisting of the amino acids phenylalanine, tryptophane and tyrosine, homotyrosine, histidine, 3-methylhistidine, histamine, dopamine and thyroxine and modifications thereof, preferably it is selected from phenylalanine, tryptophane, tyrosine and histidine, more preferably from phenylalanine, tryptophane and tyrosine, and most preferably, it is phenylalanine. X provides for localization of the dipeptide in the pocket of MHC molecules. In particular, aromatic amino acids were identified to be very suitable for an efficient localization. However, in particular for small pockets of MHC molecules, also aliphatic amino acids could represent X in the dipeptide. Therefore, in another embodiment of the invention X is selected from alanine, leucine, isoleucine, valine or methionine. In a further embodiment, X is glycine. According to the invention, Z is selected from the amino acids arginine, lysine or histidine.

The dipeptide according to the invention may be modified or unmodified. In a particularly preferred embodiment of the present invention, the dipeptide is modified. The term dipeptide further includes peptidomimetics, such as peptoids and semipeptoids which are peptide analogs, which may have, for example modifications rendering the peptide more stable. Such modifications include, but are not limited to, cyclization, N-terminus modification, C-terminus modification, peptide bond modification, including, but not limited to, CH₂-NH, CH₂-S, CH₂-S-O, O=C-NH, CH₂-O, CH₂-CH₂, S=C-NH, CH=CH or CF=CH, backbone modification and residue modification. Peptide bonds (-CO-NH-) within the dipeptide may be substituted by N-methylated bonds (-N (CH₃)-CO-), ester bonds (-C (R) H-C-O-O-C (R)-N-), ketomethylen bonds (-CO-CH₂-), o-aza bonds (-NH-N (R)-CO-),
wherein R is any alkyl, e. g., methyl, carba bonds (-CH₂-NH-), hydroxyethylene bonds (-CH (OH)-CH₂-), thioamide bonds (-CS-NH-), olefinic double bonds (-CH=CH-), retro amide bonds (-NH-CO-), peptide derivatives (-N (R)-CH₂-CO-), wherein R is the "normal" side chain, naturally presented on the carbon atom. Methods for preparing peptidomimetic compounds are well known in the art and are specified in Quantitative Drug Design, C. A. Ramsden Gd., Chapter 17.2, F. Choplin Pergamon Press (1992). The dipeptide is preferably modified on its N-terminus by at least one compound selected from the group consisting of acetyl, formyl, myristoyl, methyl or sugar group, polyethylene glycol (PEG) or dioxaoctanoic acid (Doo), and/or on its C-terminus by at least one compound selected from the group consisting of amino and methyl group, polyethylene glycol (PEG) or dioxaoctanoic acid (Doo), and/or on its side chain by at least one compound selected from the group consisting of phosphate, sulphate, methyl, ethyl, acetyl, nitro or iodo group and the like. Preferably, the dipeptide is at least modified by an acetyl group on its N-terminus and/or by an amino group on its C-terminus.

In a further embodiment according to the invention, the dipeptide is modified (solely or in addition to the modifications described above) by one or more further amino acids on its N- and/or C-terminus. The further amino acid(s) can be selected from any natural and non-natural amino acid. In particular, the further amino acid(s) is/are selected from any one of the 20 natural amino acids which are known to any skilled person in the relevant art. Such modification of the dipeptide leads to peptides having more than two amino acids, tripeptides, tetrapeptides and pantapeptides being thereby preferred.

According to a preferred embodiment of the present invention, the dipeptide is selected from the group consisting of Ac-FR-NH₂, Ac-WR-NH₂ and Ac-YR-NH₂, wherein Ac represents an acetyl group at the N-terminus and NH₂ represents an amino group at the C-terminus of the dipeptide. In another embodiment, the dipeptide is selected from any one of dipeptides No. 1 to 7 of Table 1 and 1 to 15 of Table 2. In an alternative embodiment, the dipeptide is modified in such a way that it corresponds to the peptides No. 9 to 14 of Table 1. The dipeptide No. 8 of Table 1 only shows an comperative example which does not fall into the scope of the present invention.

The present invention relates further to the use according to the invention of the dipeptide of formula XZ, alone or optionally in combination with one or more ligands, for example peptides, for the preparation of pharmaceutical compositions. In combination means that both the dipeptide and the ligand are used together e.g. for the preparation of pharmaceutical compositions. The dipeptide and the ligand may either be used as separate substances or may be in a linked form. The linkage may either be covalent or non-covalent (leading e.g. to complexes of the dipeptide(s) and ligand(s)) as described above.

In an alternative embodiment, the dipeptide can be used in combination with ligands, for example peptides, proteins or antigen-containing cell extracts, as a peptide vaccine for the preparation of vaccines, in particular peptide vaccines.

Further, in another embodiment, the MHC molecules whose load state has been changed can also be used for the preparation of pharmaceutical compositions. Those MHC molecules can be employed in isolated form or together with the cells with which they are (e.g. naturally) associated with as described above.

The present invention also provides pharmaceutical compositions. In one embodiment, the pharmaceutical compositions of the present invention typically comprise the dipeptide of formula XZ, alone or optionally in combination with one or more ligands, for example peptides, especially antigenic peptides, in particular peptides of tumor antigens or of surface proteins of pathogens, for example peptides of viral or bacterial surface proteins. The dipeptide of the invention can thereby be incorporated as an adjuvant in a pharmaceutical composition according to the invention. The adjuvant properties of the above-mentioned compounds are based on the ability of the above-mentioned compounds according to the invention to change the load state of the patient's own MHC molecules and thereby increase the activity of the ligands used. In this respect, therefore, the above-mentioned dipeptide fulfils typical adjuvant properties, which consist in further enhancing the immune response, for example to tumors or to pathogens, effected by the ligands, by, in the present case, increasing the relevant ligand load of a pharmaceutical composition according to the invention to the MHC-expressing immune cells of the treated patient. Further substances can also optionally be incorporated as adjuvants in a pharmaceutical composition according to the invention; advantageously, one or more further adjuvants will be chosen depending on the immunogenity and other properties of the antigenic ligand in the pharmaceutical composition according to the invention. In the case of weak immunogenity in particular, complete Freund's adjuvant can be used. Instead of or in combination with Freund's adjuvant, it is possible (in addition to the dipeptide) to select adjuvants from, for example, TDM, MDP, muramyl dipeptide, alum solution, CpG oligonucleotides, lipopeptides, Pam3Cys or pluronics. Finally, the antigenic ligand can also be coupled, for example chemically, to KLH (Keyhole Limpet Haemocyanin), a very immunogenic foreign protein, or alternatively KLH can be present in a pharmaceutical composition according to the invention without chemical coupling to the ligand. Furthermore, it is also possible for cytokines, in particular interferons, for example IFN-gamma, or GM-CSF, M-CSF or G-CSF, to be present as adjuvants in the pharmaceutical composition.

Such pharmaceutical compositions can be used in particular in the indications mentioned hereinbelow, especially in anti-cancer or anti-infection therapies, and can be administered to a patient *in vitro* or *in vivo.* For example, such a pharmaceutical composition according to the invention can be administered directly to a patient intravenously or subcutaneously. The change in the load state of MHC molecules preferably takes place *in vivo,* i.e. the loading with ligands, the replacement of ligands or the decrease in ligands on MHC molecules typically takes place in the patient, by administration of the pharmaceutical composition. Such pharmaceutical compositions according to the invention can likewise be administered by means of a dialysis method, in which case the change in the load state of MHC molecules likewise preferably takes place *in vivo.* As an alternative to loading *in vivo,* cells, body fluids, etc., for example from the lymph, can be taken from the patient, and a pharmaceutical composition according to the invention can be added thereto *in vitro.* The change in the load state of the MHC molecules associated with these cells, body fluids, etc. likewise preferably takes place *in vitro.* After the change in the load state of the MHC molecules *in vitro,* the cells can be returned to the patient, for example by means of a dialysis method or by intravenous or subcutaneous injection.

In one embodiment, the pharmaceutical composition is a vaccine. According to the invention, a vaccine comprises at least the dipeptide XZ and at least one ligand of an MHC molecule.

In an alternative embodiment, pharmaceutical compositions can comprise MHC molecules loaded with ligands or unloaded MHC molecules, the load state of which molecules has preferably been changed by a method according to the invention by addition of the dipeptide, and, optionally by one or more ligands. In a particular embodiment of this alternative, pharmaceutical compositions can comprise the dipeptide as well as, in addition, MHC molecules loaded with ligands or unloaded MHC molecules whose load state has been changed preferably by a method according to the invention. In another preferred embodiment of this alternative, pharmaceutical compositions can comprise the dipeptide, ligands and, in addition, MHC molecules loaded with ligands and/ or unloaded MHC molecules whose load state has been changed by a method according to the invention. Preferably used for the pharmaceutical compositions according to the invention are those MHC molecules whose load state has been changed by a method according to the invention. These are, for example, MHC molecules that have been loaded with peptides by a method according to the invention, especially with antigenic peptides, in particular peptides of a tumor antigen, or peptides that trigger an immune response under physiological conditions. The presence of ligands and/or the dipeptide together with the MHC molecules already contained in the pharmaceutical composition allows the load density of the MHC molecules in the pharmaceutical composition to be controlled by means of a corresponding adjustment of the equilibrium in the solution. This is of interest in particular when pharmaceutical compositions are not used immediately after being prepared but are first stored. Furthermore, by means of such a pharmaceutical composition, an increased change in the load state of MHC molecules can optionally be achieved *in vivo* and/or *in vitro* by means of particular (e.g. desired) ligands.

The suitable amount of the dipeptide that is to be used, optionally in combination with one or more ligands, or alternatively of the MHC molecules whose load state has been changed by a method according to the invention, can be determined by routine experiments with animal models. Such models include, without implying any limitation, rabbit, sheep, mouse, rat, dog and non-human primate models.

Pharmaceutical compositions are preferably formulated in liquid or solid form. However, any other formulation form is also possible. In liquid pharmaceutical compositions, the dipeptide is preferably present in a concentration of from 0.001 to 500 mM, preferably in a concentration of from 0.001 to 250 mM, more preferably in a concentration of from 0.001 to 100 mM. The ligands used in the pharmaceutical compositions according to the invention are typically the ligands defined previously in this description. The MHC molecule present in the pharmaceutical compositions is typically used in unloaded form or is loaded prior to use with a ligand as defined above, more preferably with peptide antigens or with fragments of such antigens, preferably having a length of from 8 to 25 amino acids, more preferably having a length of from 8 to 15 amino acids. It is likewise preferred within the scope of the subject-matter according to the invention for the MHC molecule used for the preparation of a pharmaceutical composition to be an MHC monomer or an MHC multimere as described above. The MHC monomer or MHC multimer is particularly preferably an MHC monomer or MHC multimer, or an MHC tetramer or pentamer, of MHC classes I or II as described above.

The pharmaceutical compositions of the present invention preferably comprise a safe and effective amount of the dipeptide, preferably as defined above, optionally in combination with one or more ligands as defined in the present invention, for example peptides modulating the immune response (e.g. antigenic peptides), and optionally a pharmaceutically acceptable carrier, as well as further auxiliary substances and additives. Alternatively, the pharmaceutical compositions preferably comprise a safe and effective amount of the MHC molecules whose load state has been changed by a method according to the invention, and optionally a pharmaceutically acceptable carrier, as well as further auxiliary substances and additives. As used here, "safe and effective amount" means an amount of a compound that is sufficient to significantly induce a positive modification of the condition to be treated, but that is sufficiently small to avoid serious side-effects (with a sensible ratio of advantage/risk), within the range of sensible medical discernment. A safe and effective amount of a compound will vary in connection with the particular condition to be treated, as well as the age and physical condition of the patient to be treated, the severity of the condition, the duration of the treatment, the nature of the accompanying therapy, of the particular pharmaceutically acceptable carrier used and similar factors, within the knowledge and experience of the accompanying doctor. The pharmaceutical compositions according to the invention can further be used for human and also for veterinary medical purposes.

The term "pharmaceutically acceptable carrier" used here preferably includes one or more compatible solid or liquid fillers, or diluents or encapsulating compounds, which are suitable for administration to a person. The term " acceptable ", as used here, means that the constituents of the composition are capable of being mixed with the compound and with one another in such a manner that no interaction occurs which would substantially reduce the pharmaceutical effectiveness of the composition under conventional use conditions. Pharmaceutically acceptable carriers must, of course, exhibit sufficiently high purity and sufficiently low toxicity to render them suitable for administration to a person to be treated.

The choice of a pharmaceutically acceptable carrier is determined in principle by the manner in which the pharmaceutical compositions according to the invention are administered. The pharmaceutical compositions prepared by a method according to the invention can be administered systemically. Routes for administration include transdermal, oral, parenteral, including subcutaneous or intravenous injections, topical and/or intranasal routes.

Preferred unit dose forms for injection include sterile solutions of water, physiological saline or mixtures thereof. The pH of such solutions should be adjusted to about 7.4. Suitable carriers for injection or for surgical implantation include hydrogels, devices for controlled or delayed release, polylactic acid and collagen matrices.

Suitable pharmaceutically acceptable carriers for topical application include those which are suitable for use in lotions, creams, gels and the like. If the pharmaceutical composition is to be administered perorally, tablets, capsules and the like are the preferred unit dose form. The pharmaceutically acceptable carriers for the preparation of unit dose forms which can be used for oral administration are well known in the art. The choice thereof will depend on secondary considerations such as taste, costs and storability, which are not critical for the purposes of the present invention, and can be made without difficulty by a person skilled in the art.

Some examples of compounds that can be used as pharmaceutically acceptable carriers or constituents thereof are sugars, such as, for example, lactose, glucose and sucrose; starches such as, for example, corn starch or potato starch; cellulose and its derivatives, such as, for example, sodium carboxymethylcellulose, ethylcellulose, cellulose acetate; powdered tragacanth; malt; gelatin; talc; solid lubricants, such as, for example, stearic acid, magnesium stearate; calcium sulfate; vegetable oils, such as, for example, peanut oil, cottonseed oil, sesame oil, olive oil, corn oil and oil from Theobroma; polyols, such as, for example, polypropylene glycol, glycerol, sorbitol, mannitol and polyethylene glycol; alginic acid; emulsifiers, such as, for example, the Tweens^{®}; wetting agents, such as, for example, sodium lauryl sulfate; coloring agents; flavoring agents, pharmaceutical carriers; tablet-forming agents; stabilizers; antioxidants; preservatives; pyrogen-free water; isotonic saline and phosphate-buffered solutions.

To this end, in an embodiment of the present invention, cells or tissues, preferably dendritic cells, particularly preferably maturated and non-maturated dendritic cells, can be taken from a patient beforehand, as already described above, individually or in collections of a plurality of cells, and isolated. The isolation methods used thereby have been described hereinbefore.

The MHC molecules, which are obtained by such a method and are typically located on the patient's cells (e.g. MHC bearing cells like dendritic cells, macrophages etc. as described above), can subsequently be loaded with ligands, preferably by a method according to the invention, or the ligands already present on the MHC molecule can be replaced by desired class of ligands, or the number of ligands present can be decreased or removed. Such desired classes of ligands are preferably antigens, more preferably peptide antigens, protein antigens or tumor antigens, in particular from tumor cell lysates. In a further step, the MHC molecules typically on the patient's cells bearing MHC molecules so loaded are preferably returned to the patient in a subsequent administration step, for example *via* reinjection or dialysis. The (e.g. dendritic) cells or MHC molecules so loaded by a method according to the invention then preferably trigger tumor-specific or anti-pathogenic immune responses in the patient, which lead to rejection and destruction of the transformed tissue. In a preferred embodiment of this subject-matter according to the invention, the dipeptide, in combination with one or more ligands or the MHC molecules whose load state has been changed by a method according to the invention, can be administered to a patient, preferably in the form of a pharmaceutical composition.

According to a further preferred object of the present invention, the pharmaceutical compositions according to the invention are used for the treatment of indications mentioned by way of example hereinbelow. By means of pharmaceutical compositions according to the present invention it is possible to treat, for example, those disorders or conditions which are associated with various pathologically excessive or absent immune responses. Such pharmaceutical compositions according to the invention are preferably used to trigger tumor-specific or pathogen-specific immune responses. According to another embodiment of this subject-matter according to the invention, such pharmaceutical compositions according to the present invention, which comprise, for example, the dipeptide, optionally in combination with one or more ligands, or optionally the MHC molecules loaded by a method according to the invention, preferably MHC multimeres loaded with ligands, particularly preferably MHC multimeres of class II loaded with ligands, in which the load state has been lowered or in which the ligands have been removed or replaced, can be used to attenuate aggressive immune reactions. In an embodiment that is likewise preferred, antigen-presenting cells (APC) are loaded with ligands in order to trigger, attenuate or suppress immune responses. In an even more preferred embodiment, the antigen-presenting cells are selected, for example, from endogenous or non-endogenous maturated and non-maturated dendritic cells, IDO + DC cells, B-cells or macrophages. In a further embodiment of the present invention, the MHC molecules which load state has been changed by a method according to the invention can be used for the treatment of cancer, preferably selected from colon carcinomas, melanomas, renal carcinomas, lymphomas, acute myeloid leukemia (AML), acute lymphoid leukemia (ALL), chronic myeloid leukemia (CML), chronic lymphocytic leukemia (CLL), gastrointestinal tumors, pulmonary carcinomas, gliomas, thyroid tumors, mammary tumors, prostate tumors, hepatomas, various virus-induced tumors such as, for example, papilloma virus-induced carcinomas (e.g. cervical carcinoma), adenocarcinomas, herpes virus-induced tumors (e.g. Burkitt's lymphoma, EBV-induced B-cell lymphoma), hepatitis B-induced tumors (hepatocell carcinomas), HTLV-1- and HTLV-2-induced lymphomas, acoustic neuroma, cervical cancer, lung cancer, pharyngeal cancer, anal carcinoma, glioblastoma, lymphoma, rectal carcinoma, astrocytoma, brain tumors, stomach cancer, retinoblastoma, basalioma, brain metastases, medulloblastomas, vaginal cancer, pancreatic cancer, testicular cancer, melanoma, thyroidal carcinoma, bladder cancer, Hodgkin's syndrome, meningiomas, Schneeberger disease, bronchial carcinoma, hypophysis tumor, Mycosis fungoides, esophageal cancer, breast cancer, carcinoids, neurinoma, spinalioma, Burkitt's lymphoma, laryngeal cancer, renal cancer, thymoma, corpus carcinoma, bone cancer, non-Hodgkin's lymphomas, urethral cancer, CUP syndrome, head/neck tumors, oligodendroglioma, vulval cancer, intestinal cancer, colon carcinoma, esophageal carcinoma, wart involvement, tumors of the small intestine, craniopharyngeomas, ovarian carcinoma, abdomen tumors, ovarian cancer, liver cancer, pancreatic carcinoma, cervical carcinoma, endometrial carcinoma, liver metastases, penile cancer, tongue cancer, gall bladder cancer, leukemia, plasmocytoma, uterine cancer, lid tumor, prostate cancer, etc., or for the treatment of infectious diseases selected from influenza, malaria, SARS, yellow fever, AIDS, Lyme borreliosis, Leishmaniasis, anthrax, meningitis, viral infectious diseases such as AIDS, Condyloma acuminata, hollow warts, Dengue fever, three-day fever, Ebola virus, cold, early summer meningoencephalitis (FSME), flu, shingles, hepatitis, herpes simplex type I, herpes simplex type II, Herpes zoster, influenza, Japanese encephalitis, Lassa fever, Marburg virus, measles, foot-and-mouth disease, mononucleosis, mumps, Norwalk virus infection, Pfeiffer's glandular fever, smallpox, polio (childhood lameness), pseudo-croup, fifth disease, rabies, warts, West Nile fever, chickenpox, cytomegalic virus (CMV), bacterial infectious diseases such as abort (prostate inflammation), anthrax, appendicitis, borreliosis, botulism, Camphylobacter, Chlamydia trachomatis (inflammation of the urethra, conjunctivitis), cholera, diphtheria, donavanosis, epiglottitis, typhus fever, gas gangrene, gonorrhoea, rabbit fever, Helicobacter pylori, whooping cough, climatic bubo, osteomyelitis, Legionnaire's disease, leprosy, listeriosis, pneumonia, meningitis, bacterial meningitis, anthrax, otitis media, Mycoplasma hominis, neonatal sepsis (Chorioamnionitis), noma, paratyphus, plague, Reiter's syndrome, Rocky Mountain spotted fever, Salmonella paratyphus, Salmonella typhus, scarlet fever, syphilis, tetanus, tripper, tsutsugamushi disease, tuberculosis, typhus, vaginitis (colpitis), soft chancre and infectious diseases caused by parasites, protozoa or fungi, such as amoebiasis, bilharziosis, Chagas disease, Echinococcus, fish tapeworm, fish poisoning (Ciguatera), fox tapeworm, athlete's foot, canine tapeworm, candidosis, yeast fungus spots, scabies, cutaneous Leishmaniosis, lambliasis (giardiasis), lice, malaria, microscopy, onchocercosis (river blindness), fungal diseases, bovine tapeworm, schistosomiasis, sleeping sickness, porcine tapeworm, toxoplasmosis, trichomoniasis, trypanosomiasis (sleeping sickness), visceral Leishmaniosis, nappy dermatitis, miniature tapeworm, for the treatment of autoimmune diseases, such as, for example, autoimmune disorders, in particular type I autoimmune disorders or type II autoimmune disorders or type III autoimmune disorders or type IV autoimmune disorders, such as multiple sclerosis (MS), rheumatoid arthritis, diabetes, type I diabetes, systemic lupus erythematosus (SLE), chronic polyarthritis, Basedow's disease, autoimmune forms of chronic hepatitis, Colitis ulcerosa, type I allergic disorders, type II allergic disorders, type III allergic disorders, type IV allergic disorders, fibromyalgia, hair loss, Bechterew's disease, Crohn's disease, Myasthenia gravis, neurodermitis, Polymyalgia rheumatica, progressive systemic sclerosis (PSS), psoriasis, Reiter's syndrome, rheumatic arthritis, psoriasis, vasculitis, etc.

The present invention relates further to the use of MHC molecules (either isolated or as integral component of the cell membrane of cells, e.g. a membrane fraction of cells or as component of living cells), which can be prepared by a method according to the invention, for the preparation of a pharmaceutical composition for the treatment of the above-mentioned indications.

The present invention likewise preferably relates to the use of the dipeptide, optionally in combination with one or more ligands, or alternatively of MHC molecules whose load state has been changed by a method according to the invention, preferably MHC multimeres, for screening methods and diagnostic methods for seeking and identifying new antigens, in particular tumor antigens, pathoantigens and autoantigens, and for detecting specific T-cells, for example autoreactive T-cells or cytotoxic T-cells, or for monitoring a specific T-cell response. Typically, to this end, in a first step there is provided a composition comprising the dipeptide, optionally in combination with one or more ligands, or alternatively comprising MHC molecules whose load state has been changed by a method according to the invention. MHC molecules are MHC molecules as defined above. The compositions used here can correspond to the compositions provided in the methods for changing the load state of MHC molecules. The MHC molecules used in the present case can be loaded with ligands or unloaded. The ligands preferably correspond to the ligands defined hereinbefore in the description and are chosen specifically depending on the underlying object. Preferably, the ligands loaded onto MHC molecules are those which lead to an immune response also under physiological conditions. The ligands used and/or identified in the screening method or diagnostic method are preferably ligands as defined above of MHC molecules, more preferably ligands of tumor- or pathogen-specific antigens as well as antigens of autoreactive T-cells. For example, by the targeted binding of antigens to MHC molecules by means of the screening or diagnostic method according to the invention, the formation of specific T-cell receptors or T-cells on that antigen in a patient can be checked. The immune response optionally obtained with the screening can then be brought into direct association with an indication. Typically, by a method according to the invention, ligands are loaded onto the MHC molecule in such an amount that the amount of ligands on the MHC molecules is sufficient to stimulate an immune response, preferably a T-cell response. In a further step, the MHC molecule can be bound to a solid phase or to a matrix. A solid phase or a matrix within the scope of the present method is preferably any surface to which MHC molecules can be bound directly, *via* a linker, *via* labelling or *via* their affinity. The MHC molecules can be labelled as described above for the binding to a solid phase. For the binding to a solid phase or to a matrix, suitable buffers can be added to the composition. Suitable buffers are known to a person skilled in the art. Suitable buffers within the scope of the present method are, for example, PBS buffer, BSA-PBS buffer (PBS buffer with bovine serum albumin (BSA)), FCS-PBS buffer (PBS buffer with fetal calf serum (FCS)), phosphate buffer, TRIS-HCl buffer, tris-phosphate buffer, or further suitable buffers. The concentrations and amounts of the buffers to be used preferably correspond to those mentioned hereinbefore in the description. In a further step, a substance to be investigated, for example a body fluid or a homogenized tissue, preferably blood or tissue, optionally in one of the buffers described hereinbefore, can be added. The physiological binding partners contained in the added substance conventionally bind to the immobilized MHC molecules optionally loaded with ligands by a method according to the invention. After binding of the physiological binding partners that are present to the MHC molecules, the solid phase or matrix is conventionally washed with a buffer, preferably with one of the buffers described hereinbefore. In a particularly preferred embodiment, the physiological binding partner is preferably a T-cell, particularly preferably a T-cell that possesses a high affinity for an antigen, yet more preferably a T-cell having a high affinity for a tumor- or pathogen-specific antigen. Likewise particularly preferably, the immune response produced in a screening method or to be detected in a diagnostic method is an antigen-specific T-cell response. In a final step, the physiological binding partners, bound to the MHC molecules, of the MHC molecules optionally loaded with ligands can be identified and isolated by a biophysical or biochemical detection or isolation method as described above, preferably by means of FACS or MACS. According to an embodiment that is likewise preferred, T-cells can be isolated antigen-specifically by means of MACS in such method using MHC class II-carrying magnetic beads. The steps mentioned in the above-mentioned screening method or diagnostic method can be changed in any desired sequence. Screening methods or diagnostic methods according to the present invention preferably include *in vitro* T-cell assays, particularly preferably proliferation assays, ELISPOTS, ELISA methods, chromium release assays, high-throughput screening methods (HTS), etc.

A particular embodiment of the screening or diagnostic method according to the invention relates to a method for seeking and identifying new tumor antigens, for detecting specific cytotoxic T-cells or for monitoring a specific T-cell response, comprising the following steps:
a) providing a composition comprising MHC molecules whose load state has been changed by a method according to the invention; and
b) determining the interaction of MHC molecules whose load state has been changed by a method according to the invention, with a physiological binding partner of the MHC molecules by means of a biochemical or biophysical detection method; and
c) optionally identifying and isolating the physiological binding partner of the MHC molecules whose load state has been changed by a method according to the invention, by means of a biochemical or biophysical detection or isolation method.

In a further embodiment of the present invention, in a screening method or diagnostic method, the MHC molecules whose load state has been changed by a method according to the invention, preferably MHC multimeres, can be used for the antigen-specific identification of (e.g. autoreactive) T-cells, preferably of tumor-specific, pathogen-specific or autoreactive T-cells. There are used as ligands preferably those antigens which are associated with one of the indications mentioned hereinbefore. Such a diagnostic method is typically carried out *ex vivo,* for example by adding samples from the patient, for example samples of body fluids, in particular blood or lymph samples, to MHC molecules loaded according to the invention with ligands (for example derived from a pathogen antigen or a tumor antigen, for example a fragment thereof) by means of the dipeptide. In a subsequent step, the binding of cells in the patient's sample, for example (auto)reactive T-cells, to the loaded, preferably tetrameric, MHC molecules is detected, for example by means of suitable detection methods, in particular by labelling of the MHC molecules and/or ligands, for example by fluorescent labelling or other spectroscopically detectable compounds. The detection can be carried out in particular by means of FACS analysis.

Finally, the present invention also relates to a method of identifying substances having the property of changing the load state of MHC molecules. Such a method can be carried out using both a loaded or an unloaded MHC molecule and can be based, for example, on a kinetic measurement of the ligand dissociation and/or ligand binding or on the detection of the underlying conformational changes. Effects that are to be observed experimentally of substances that may change the load state then consist in a method according to the invention for the determination thereof in, for example, an accelerated dissociation of ligands bound to MHC and/or accelerated binding of ligands, in particular peptides, added to unloaded MHC molecules. However, spectroscopic or thermodynamic measuring methods, in particular in conjunction with conformation-specific antibodies, can also be used for determining the activities of test substances and hence for the identification thereof.

If the method according to the invention is to be carried out on the basis of a loaded MHC molecule (embodiment 1), such a method according to the invention for determining substances that change the MHC load, in particular MHC II load, is generally obtained from a method step (a) provision of MHC molecules loaded with ligands, for example in solution or on a surface, for example a metal surface, in particular a gold surface, (b) addition of a dipeptide of formula XZ to be tested, and (c) measurement of the dissociation of the ligands originally located on the MHC molecules. The measurement can be carried out, for example, as mentioned above, by kinetic measurement, or alternatively by concentration measurement of the dissociated ligands and/or of the ligands remaining on the MHC molecules. The concentration measurement can be carried out by appropriate spectroscopic methods (e.g. surface plasmon resonance, which is able to detect the difference between loaded and unloaded MHC molecule) or by, for example, spectroscopically detectable changes between the loaded and unloaded MHC molecules (e.g. changed fluorescence or absorption properties of the bound or non-bound ligand in the complex or of the complex). It is also possible to use microcalorimetric methods or other methods known to the person skilled in the art, in order to detect the changes brought about by the addition of the test substances in method step (b), compared with the initial state.

In the case of a procedure for determining suitable substances without a load (embodiment 2), the method is as follows: in a first method step (a), unloaded MHC molecules, for example in solution or on a surface, for example a metal surface, in particular a gold surface, are provided; in a method step (b1), the dipeptide of formula XZ to be tested is added; and in a method step (b2), a ligand of the MHC molecule is added. Method steps (b1) and (b2) can also be carried out simultaneously, i.e. in such a case a mixture of ligand and test substance is added. In a final method step (c), the loading of the MHC molecules with the ligand is measured by the methods already mentioned above, for example by kinetic measurements, evaluation preferably being made by comparison with the experimental results obtained without addition of the test substance. Suitable test substances can in turn be specified as a result.

In a variant of the two above-mentioned embodiments of the method according to the invention for identifying substances having the property of changing the load state of MHC molecules, two or more different test substances, typically in a mixture, can be added to a test batch. This permits a higher throughput of test substances. The one or more test substance(s) that has/have positive activity in a positive test batch must then be identified in a subsequent method step. In another variant, methods according to the invention can be carried out in the manner of a competition assay. To this end, embodiment 1, for example, is so modified that in method step (b) thereof, not only the test substance is added but, before, at the same time as or after the addition of the test substance, a second ligand for the MHC molecule is added to the test batch. Then, typically by means of the above-described methods, for example the concentration of the second ligand on the MHC molecules or the kinetics of the binding of the second ligand to the MHC molecule is determined. The activity of the test substance is therefore in turn derived from a comparison of the tests without addition of the test substance and after addition of the test substance.

### Description of the Figures

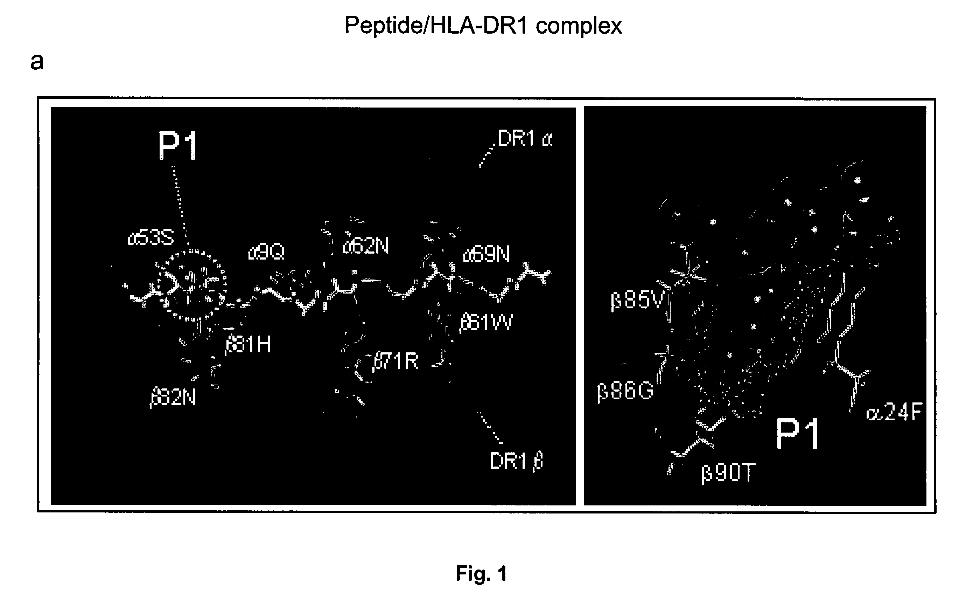

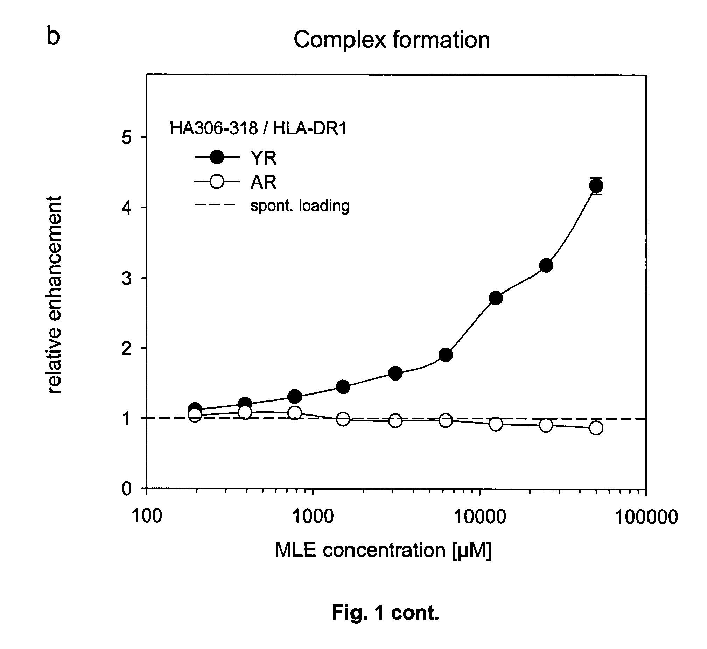

Fig. 1 shows that amino acid side chains act as 'MHC-loading enhancers' (MLE). 1 a depicts a P1 pocket as MLE target site. Left panel is a top view on the peptide binding site of the class II MHC molecule indicating the approximate position of the P1 pocket. Only the α₁-(blue ribbon) and the α₂-domain (red ribbon) are depicted. Location of the backbone of a peptide ligand is indicated in magenta; N- and C-terminal site are labelled. Right panel is a side view of the P1 pocket loaded with the tyrosine side chain of a peptide ligand. Surface of the pocket is indicated in yellow; amino acid residues forming this pocket are indicated. The peptide ligand is shown in spacefill mode. Images are based on the crystal structure of HA306-318/HLA-DR1 (PDB: 1DLH). 1b illustrates the catalytic impact of dipeptides on complex formation. The complex formation between HA306-318 and soluble HLA-DR1 was carried out in the presence of titrated amounts of the dipeptides YR (filled circle) or AR (open circle) or in the absence of these dipeptides (dashed line). Complex formation was determined by ELISA and is expressed as relative enhancement in reference to the spontaneous complex formation in the absence of any dipeptide catalyst.

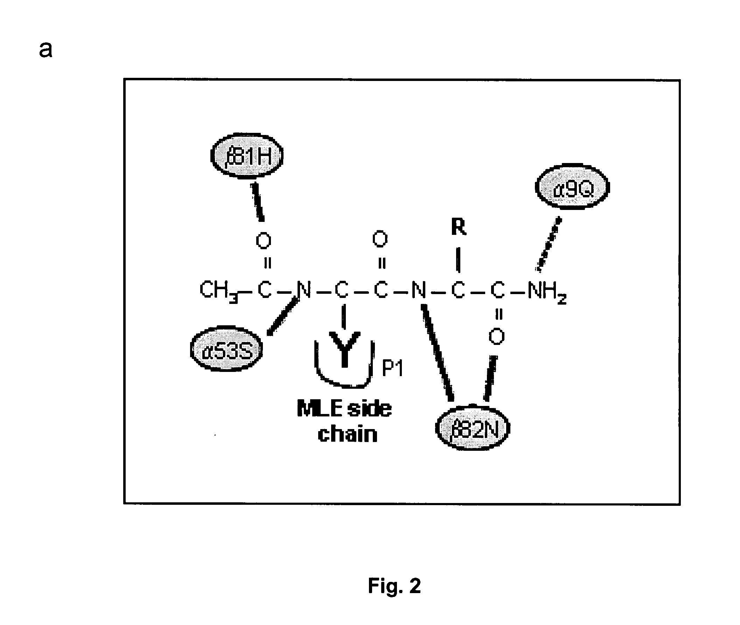

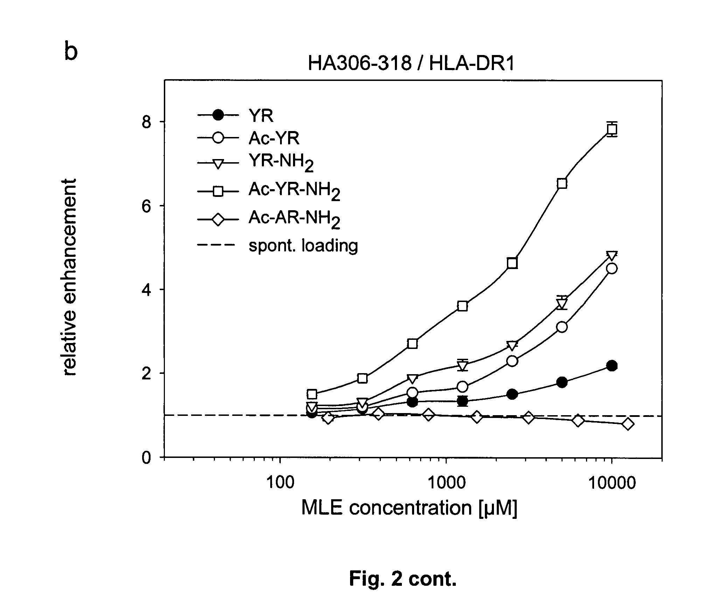

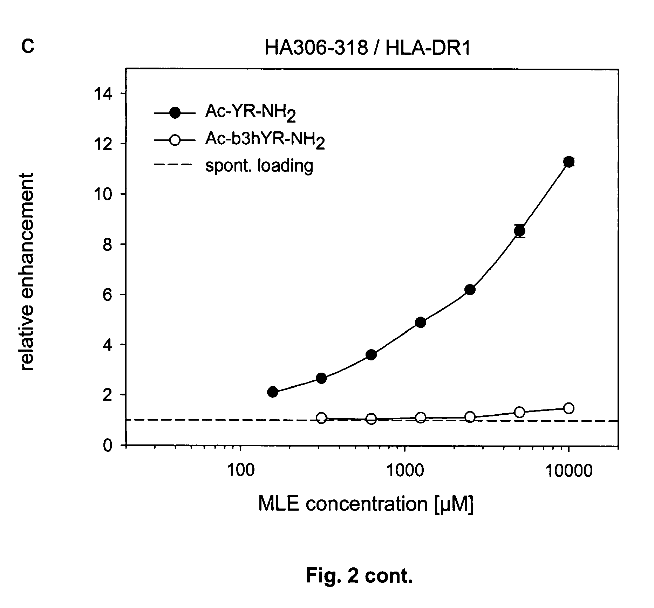

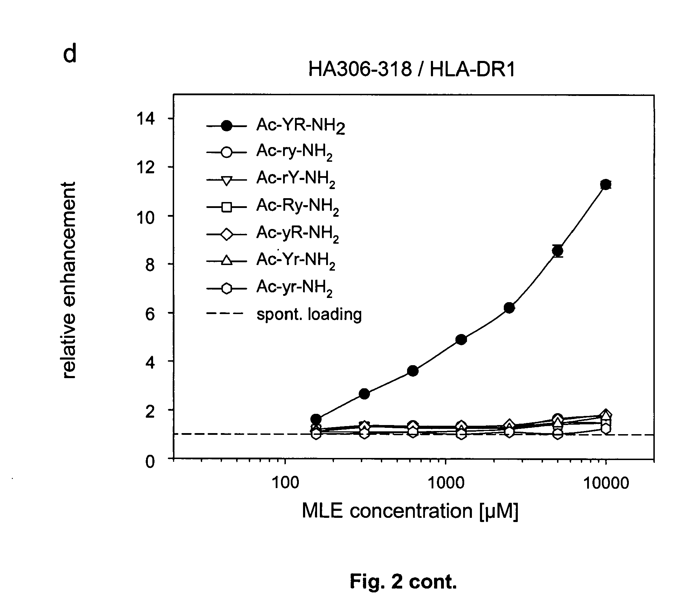

Fig. 2 illustrates the catalytic activity of YR-derivatives. 2a illustrates H-bond interactions proximal to the P1 pocket. A schematic view of H-bond interactions between the peptide backbone and residues within the MHC binding site is shown. H-bonds are depicted as dashed blue lines; residues of the MHC binding site acting as H-bond donor/receptor are indicated by blue elliptic circles. The anchor amino acid (Y) acting as MLE side chain is indicated. H-bond interactions are based on the crystal structure of HA306-318 / HLA-DR1 (PDB: 1DLH). 2b shows the impact of the H-bond network on the catalytic activity of dipeptides. To provide interaction partners for the natural H-bond network N-terminal acetylation and C-terminal amidation was introduced to the YR dipeptide. The influence of these modifications on the catalytic activity is shown for the complex formation between HA306-318 and HLA-DR1. The loading reaction was carried out in the presence of titrated amounts of YR (filled circle), Ac-YR (open circle), YR-NH₂ (open triangle), Ac-YR-NH₂ (open square) and as control Ac-AR-NH₂ (open diamond). The experiment was carried out as described in Fig. 1b; the spontaneous load in the absence of any dipeptide catalyst is indicated by a dashed line. 2c and d demonstrate the impact of backbone spacing and chiral arrangement of a dipeptide. 2c shows the relative enhancement of complex formation by a dipeptide derivative in which the backbone spacing, the dipeptide was increased by using the L-homotyrosine (b3hY) instead of tyrosine (Y). The relative enhancement is shown for the formation of HA306-318/HLA-DR1 in the presence of titrated amounts of Ac-b3hYR-NH₂ (open circle) or Ac-YR-NH₂ (filled circle). In 2d, the influence of D-amino acids is shown. Complex formation of HA306-318/HLA-DR1 was carried out in the presence of titrated amounts of Ac-YR-NH₂ (filled circle), Ac-ry-NH (open circle), Ac-rY-NH₂ (open triangle), Ac-Ry-NH₂ (open square), Ac-yR-NH₂ (open diamond), Ac-Yr-NH₂ (open triangle up), Ac-yr-NH₂ (open hexagon). L-amino acids are indicated by capital letters, D-amino acids by small letters.

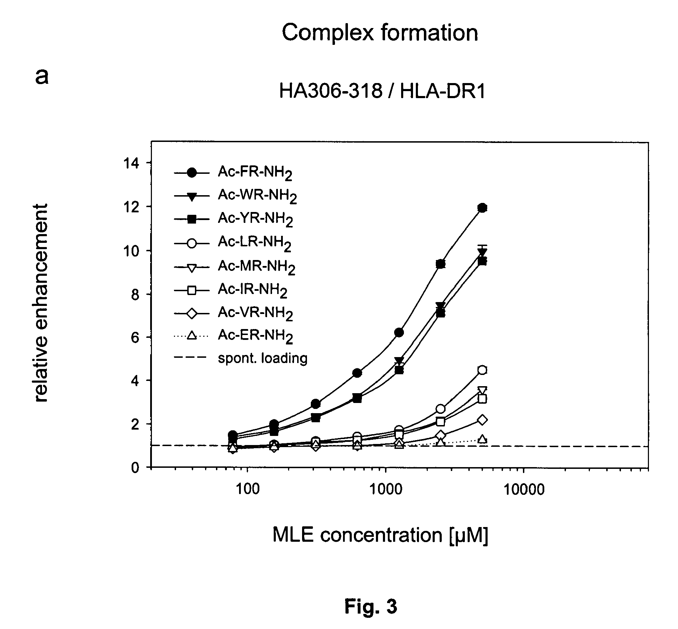

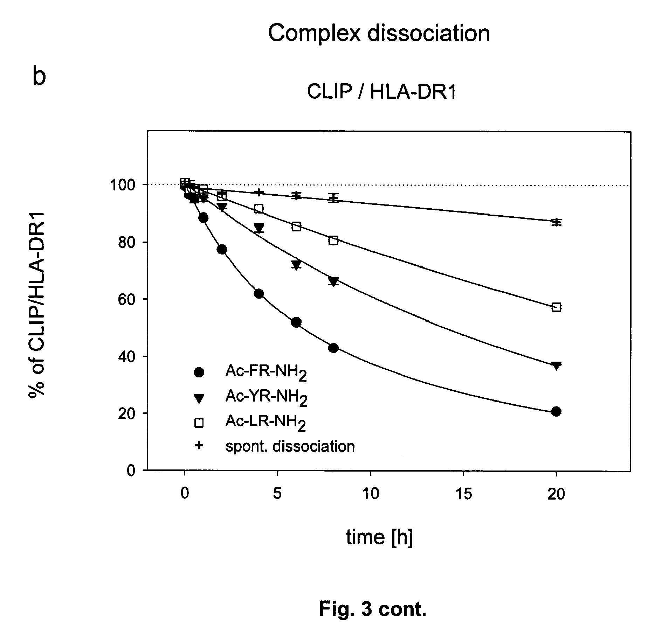

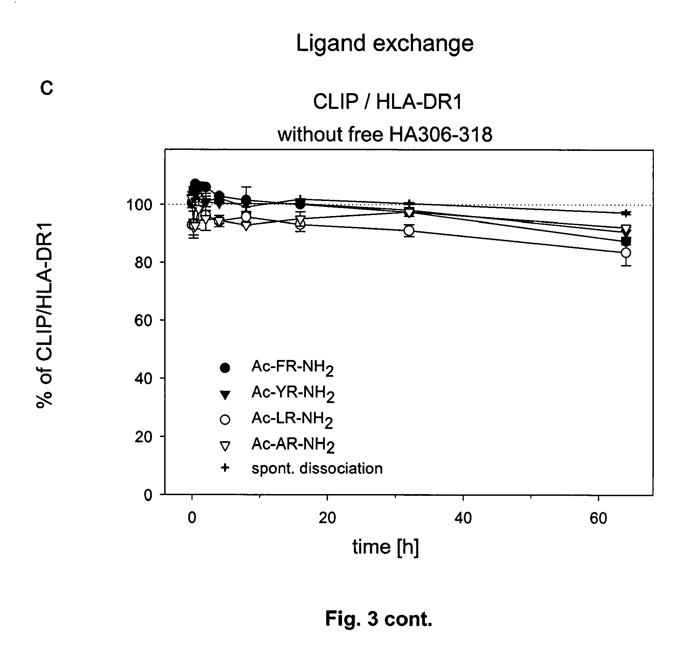

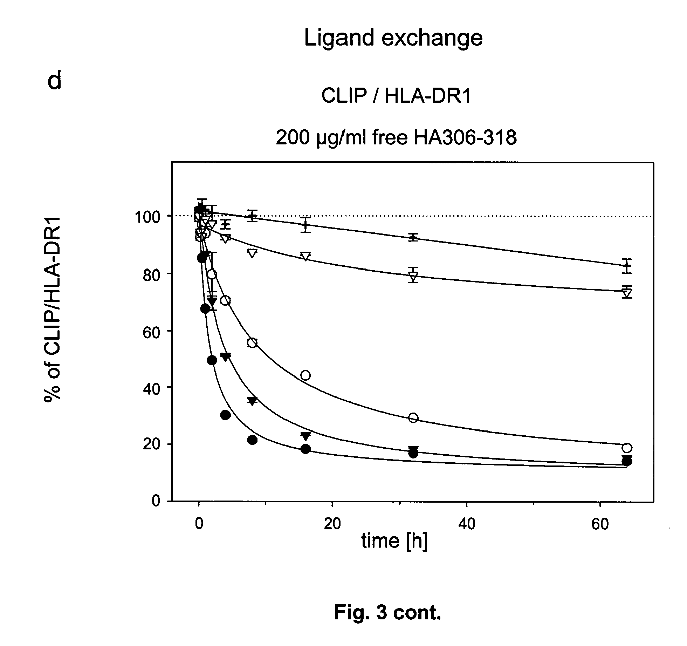

Fig. 3 illustrates the impact of peptide-MLE on complex formation, complex dissociation and ligand exchange. 3a shows the complex formation. The relative enhancement of complex formation of HA306-318/HLA-DR1 is shown for the dipeptide derivatives Ac-FR-NH₂ (filled circle), Ac-WR-NH₂ (filled triangle down), Ac-YR-NH₂ (filled square), Ac-LR-NH₂ (open circle), Ac-MR-NH₂ (open triangle down), Ac-IR-NH₂ (open square), Ac-VR-NH₂ (open diamond), Ac-ER-NH₂ (open triangle up). The experiment was carried as shown in Figure 1 b with titrated amounts of dipeptides. 3b shows the complex dissociation. A complex dissociation of CLIP/HLA-DR1 was determined in the presence of 10 mM Ac-FR-NH₂ (open circle), AC-YR-NH₂ (filled triangle), Ac-LR-NH₂ (open square) and in the absence of any dipeptide (cross). At indicated time points the percentage of remaining CLIP/HLA-DR1 complex was determined by ELISA. Complex dissociation was carried out in the presence of 200 µg/ml free HA306-318. 3c und d illustrate the ligand exchange. To demonstrate that peptide-MLE catalyze ligand exchange, the dissociation experiment was carried out both, in the presence of free HA306-318 (3d) as well as in the absence of any free HA306-318 (3c). The experiment was carried out as in Fig. 3b using 10 mM Ac-FR-NH₂, (filled circle), Ac-YR-NH₂ (filled triangle), Ac-LR-NH₂ (open circle), Ac-AR-NH₂ (open triangle) and in the absence of any dipeptide catalyst (cross).

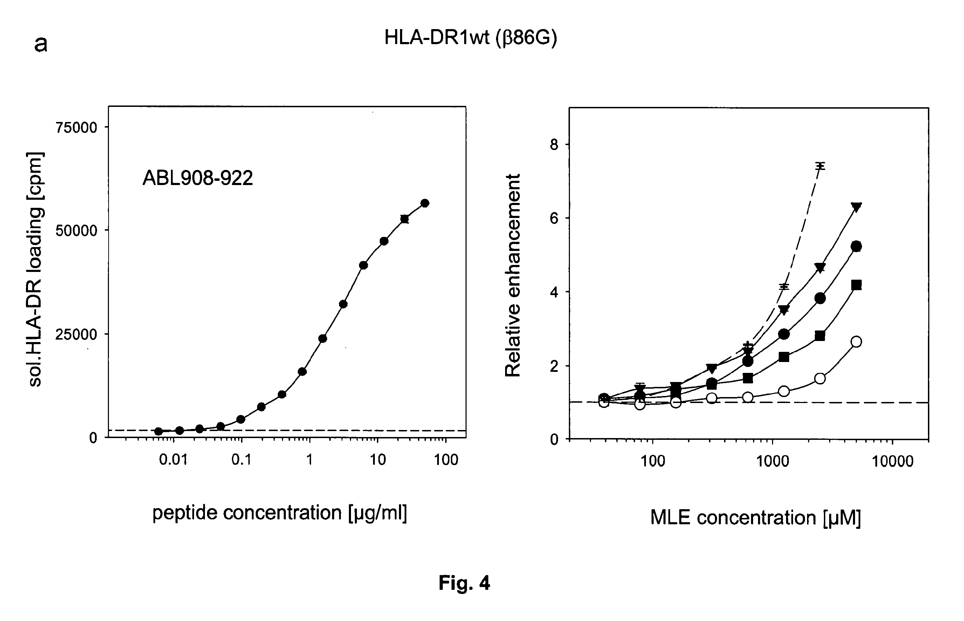

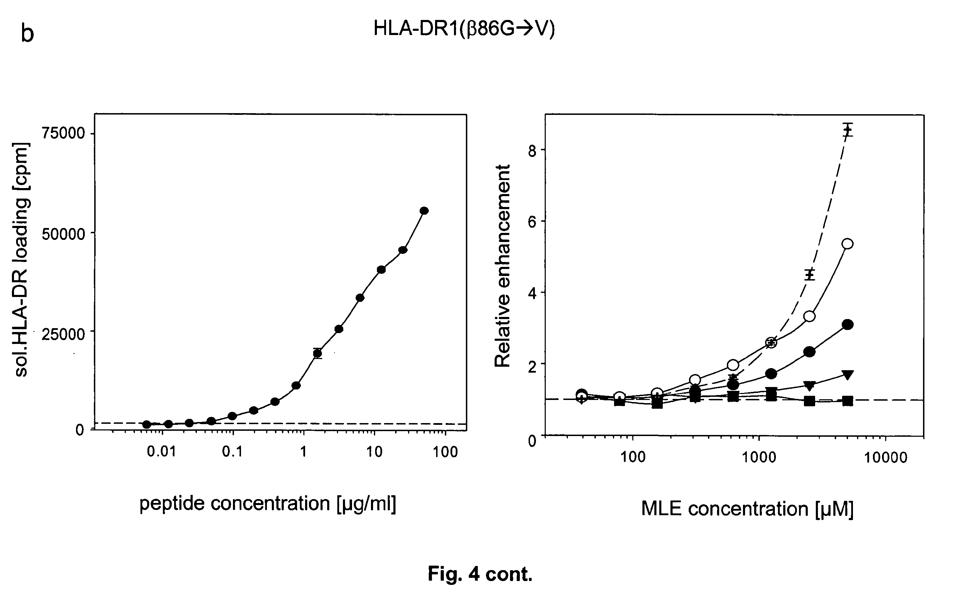

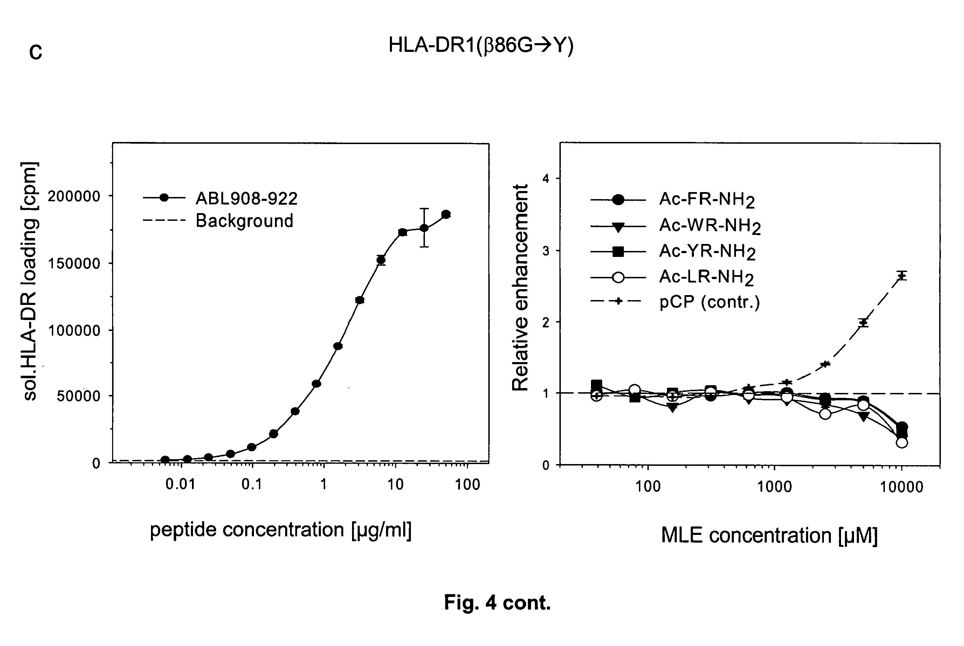

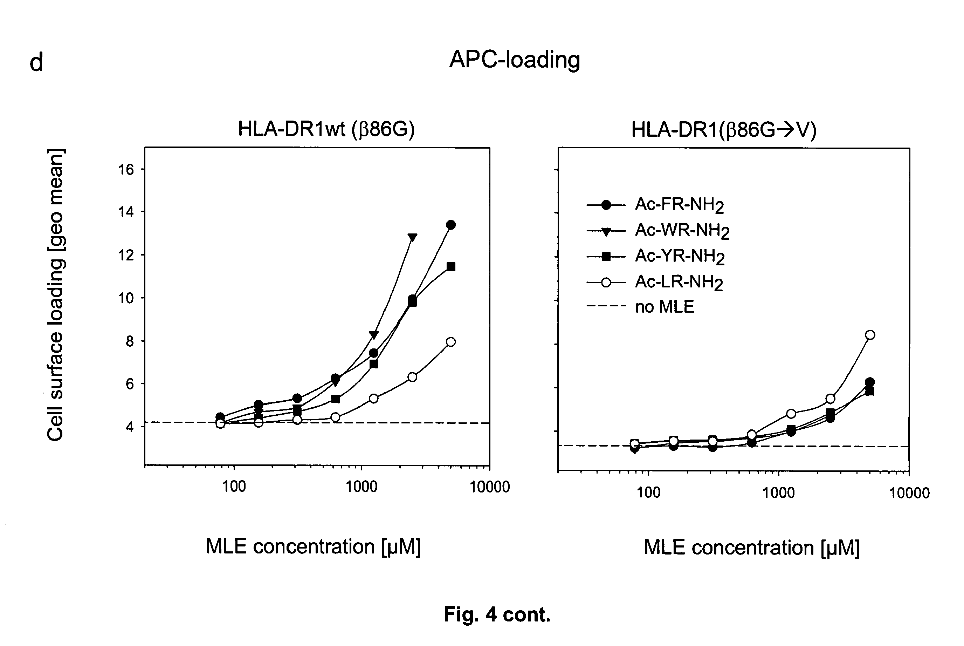

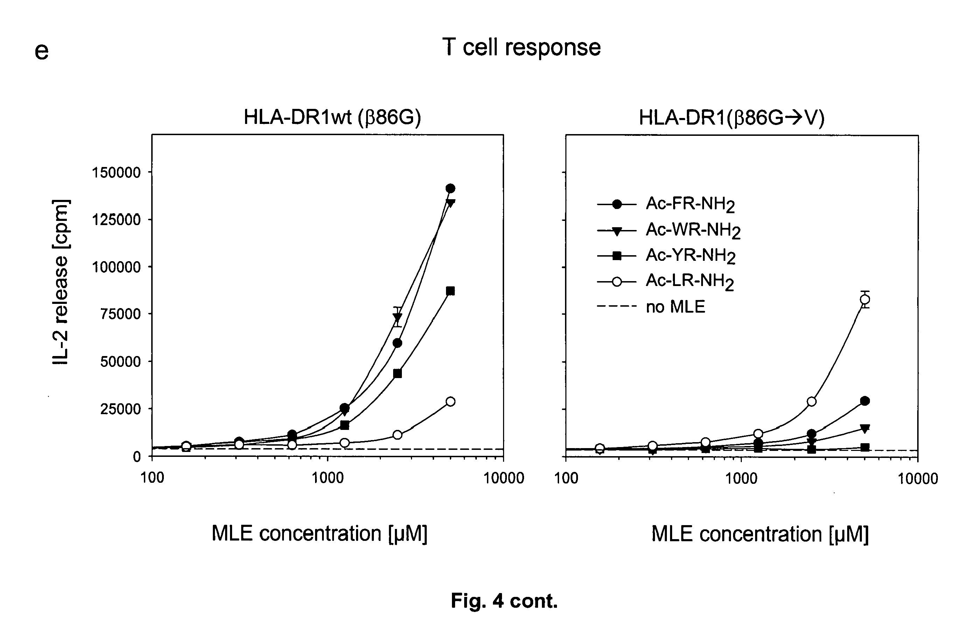

Fig. 4 depicts the allele specificity of peptide-MLE. 4a-c show the influence of P1 pocket mutations. Recombinant soluble HLA-DR1 molecules were mutated at position 86 and used in loading experiments with ABL908-920. The loading was determined by ELISA. In the left panels the spontaneous loading is shown for wt HLA-DR1 (β86G) (4a) and for the two mutants HLA-DR1 (β86G→V) (4b) and HLA-DR1 (β86G→Y) (4c). Loading was carried out for 1h with indicated amounts of biotinylated ABL908-920. The formation of ABL908-920/HLA-DR complex is expressed in counts per minute (cpm); dashed line indicates background signal. In the right panels, the influence of titrated amounts of Ac-FR-NH₂ (filled circle), Ac-WR-NH₂ (filled triangle down), Ac-YR-NH₂ (filled square) and Ac-LR-NH₂ (open circle) is shown for each figure. As control p-chlorophenol (pCP; bold with dashed line), an organic MLE compound acting independently of P1, was included. Complex formation is expressed as relative enhancement in reference to the spontaneous complex formation in the absence of any dipeptide catalyst. 4d shows the APC loading and 4e shows the T cell response. APC loading is shown in the upper panels. Fibroblast cells expressing either wild type HLA-DR1 (left panel) or HLA-DR1 molecules in which the residue β86G was substituted by V (right panel), were incubated with biotinylated ABL908-922, a peptide able to bind to both variants of HLA-DR1. The loading was carried out in the presence of titrated amounts of Ac-FR-NH₂ (filled circle), Ac-WR-NH₂ (filled triangle), Ac-YR-NH₂- (filled square), Ac-LR-NH₂ (open circle) or in the absence of any peptide-MLE. The amount of ABL908-922 loaded onto the cell surface was determined by FACS and is expressed as geo. mean. T cell response is shown in the lower panels. Fibroblasts loaded according to Fig. 4d were used to stimulate the ABL908-922-specific T cell hybridoma SaABL1/G2. The IL-2 release is indicated, dashed lines represent the T cell response triggered in the absence of any MLE compound.

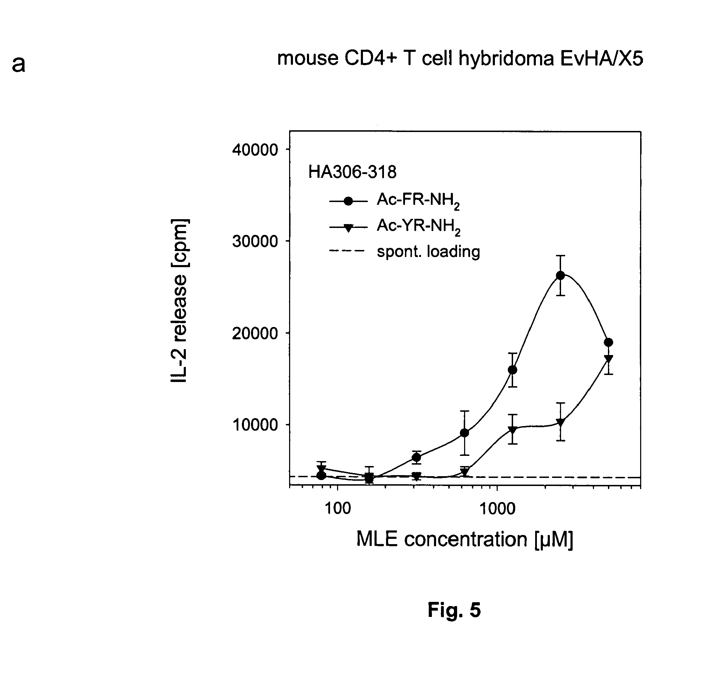

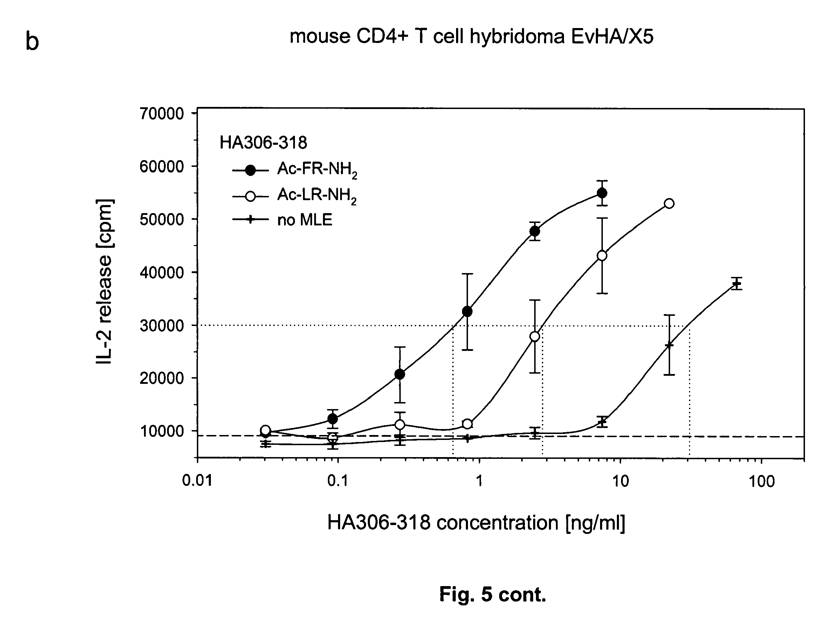

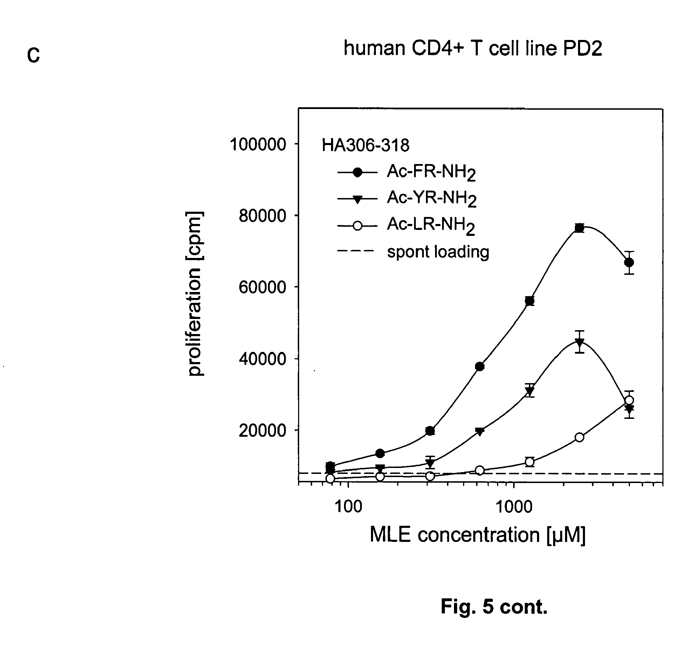

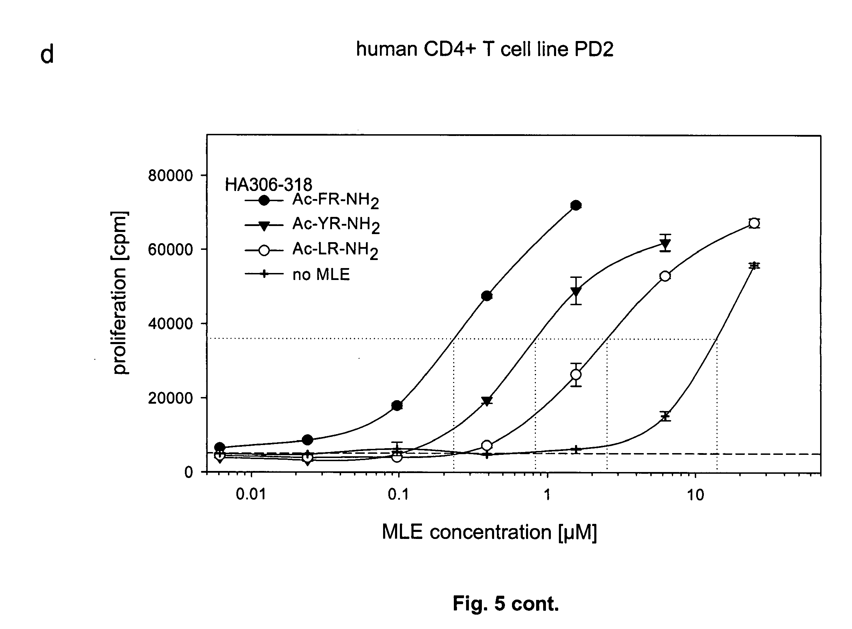

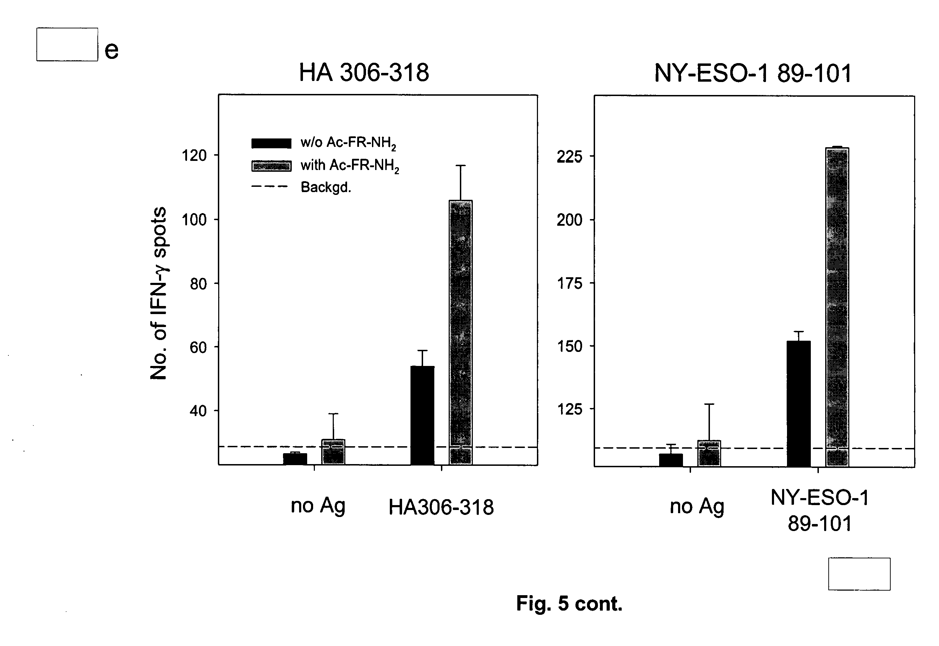

Fig. 5 illustrates the amplification of the antigen-specific T cell response by peptide-MLE. The influence of peptide-MLE on the antigen-specific CD4+ T cell response was tested with a mouse T cell hybridoma EvHA/X5 (5a and b) and a human T cell line PD2 (5 c and d). Both recognize the HA306-318 antigen in the context of HLA-DR1. 5a and c show the enhancement of the T cell response in the presence of titrated amounts of the peptide-MLE Ac-FR-NH₂ (filled circle), Ac-YR-NH₂ (filled triangle) or Ac-LR-NH₂ (open circle) in the presence of 15 ng/ml HA306-318 for EvHA/X5 and 2 ng/ml HA306-318 for PD2. The response in the absence any MLE is indicated as a dashed line. 5b and d are showing the dose response curves of HA306-318 in the presence of 5 mM Ac-FR-NH₂ (filled circle) or Ac-LR-NH₂ (open circle), 3mM Ac-YR-NH₂ (filled triangle) or in the absence of any peptide-MLE (cross). Dashed line indicates the background.

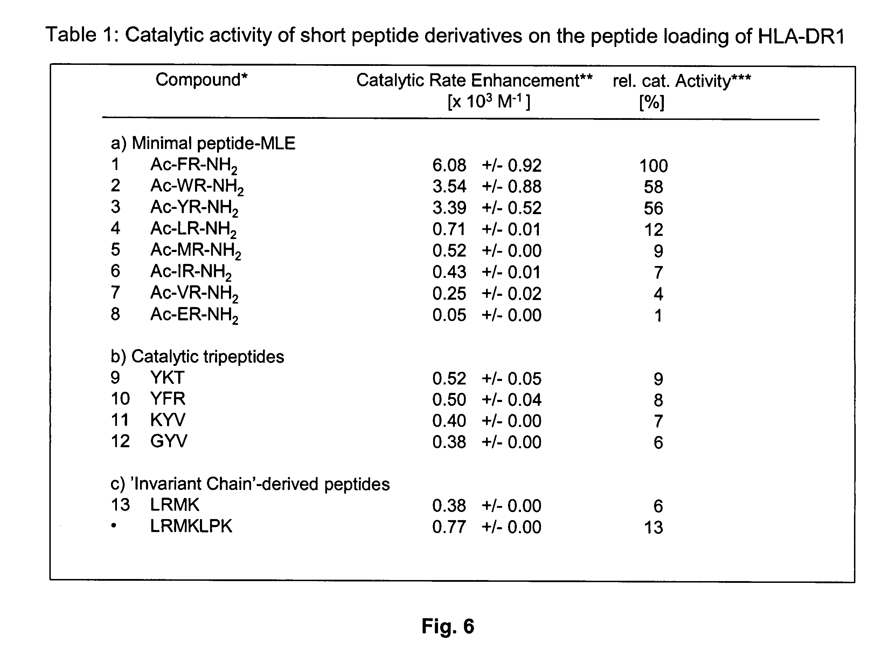

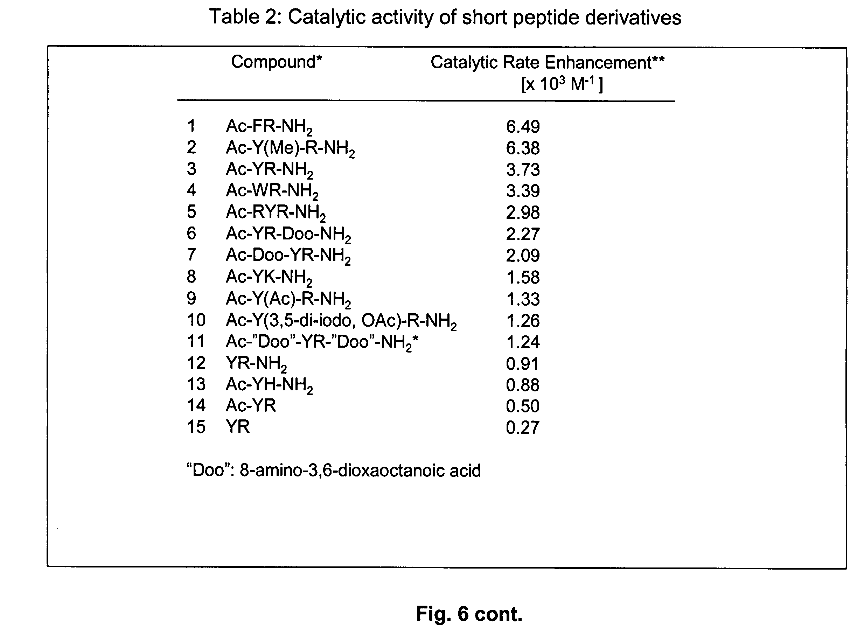

Fig. 6 shows the catalytic activity of short peptide derivatives on the peptide loading of HLA-DR1 (Table 1) and binding to soluble HLA-DR1 (Table 2).

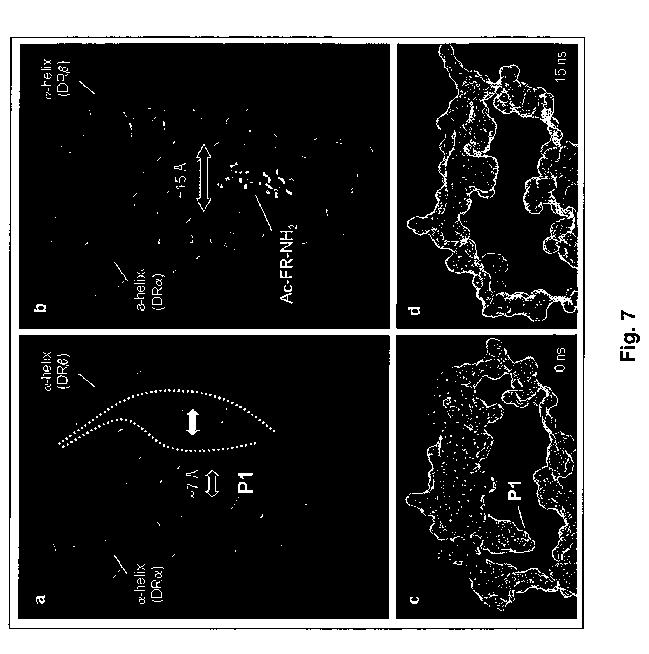

Fig. 7 shows a model of stabilization of the receptive conformation. That figure shows an overlay of the HLA-DR molecule conformation before and after a molecular dynamic (MD) calculation covering 15 ns. The calculation was carried out, either with an empty HLA-DR molecule (left panel) or an HLA-DR1 molecule, in which the peptide-MLE Ac-FR-NH₂ was docked into the P1 pocket prior to the MD calculation.

### Advantages of the invention

Class II MHC molecules are peptide-receptors displaying antigens for the surveillance by CD4+ T cells. Loading of the MHC molecule requires the transition into the peptide receptive state, a conformation stabilized by the chaperone HLA-DM. While the activity of HLA-DM is restricted to the acidic endosomal loading compartment, it could recently be demonstrated that small organic compounds can achieve the same effect also on cell surface MHC. Herein, it is shown, that also the anchor amino acids of short peptide derivatives trigger ligand exchange and antigen-loading. The effect was evident both on soluble and on the cell surface HLA-DR molecules and enhanced the sensitivity of T cell responses up to 50fold. The activity of dipeptides correlated with the allele-specific anchor preferences of HLA-DR molecules, indicating that, analogue to organic 'MHC-loading enhancer' (MLE), also here occupation of pocket P1 of the MHC molecule mediated the effect. The catalytic activity was strictly stereospecific and could be further increased by modifications offering interactions with the conserved H-bond network proximal to the P1 pocket. Molecular dynamics calculations suggest a key role of the P1-pocket in the transition to the non-receptive state. Within few nanoseconds 'empty' HLA-DR molecules become non-accessible by 'closing' the P1 pocket, an effect that '*in silico*' was prevented by the peptide-MLE.

The present data show that not only 'exotic' chemicals such as adamantyl compounds but also short peptide fragments can mediate the effect. 'P1-Anchor' side chains of dipeptides can provoke both ligand exchange and antigen loading, suggesting that amino acid derivatives and other natural polypeptides may directly influence the antigen capture by cell surface MHC molecules.

It was previously reported that the antigen repertoire displayed by human class II MHC molecules can be affected by small organic compounds. Analogue to HLA-DM these compounds stabilize the peptide receptive state of HLA-DR molecules resulting in accelerated antigen-loading and ligand exchange. In contrast to HLA-DM these compounds are active also at neutral pH. They can therefore affect the MHC molecules directly at the cell surface by catalyzing the removal or loading of CD4+ T cell antigens. A key-role in this process seems to play the pocket P1.

The P1 pocket is located at the end of the binding side close to the N-terminal side of the peptide ligand (Fig. 1a). The pocket accommodates the first anchor residue of the ligand and is largely conserved among HLA-DR molecules. However, a dimorphism at position β86, which restricts the depth of the hydrophobic pocket, divides the family into two subgroups. Allelic HLA-DR variants containing β86V have shallow pocket that accommodates only small aliphatic amino acids, while the deeper β86G containing pocket binds preferentially the bulkier aromatic amino acids.

Studies in which adamantyl derivatives acted as 'MHC-loading enhancer' (MLE) revealed that the occupation of P1 by this compound mediated ligand exchange. It was therefore speculated that a similar effect could also be achieved with amino acid side chains. To test this hypothesis soluble HLA-DR1 (DRA*0101, DRB1*0101) was incubated with the high-affinity peptide ligand HA306-318 in the absence of amino acids or short dipeptides. DRB1*0101 forms a β86G pocket, preferring the aromatic anchor amino acids. While free amino acids did not exhibit any effect (data not shown) the YR dipeptide consisting of tyrosine (Y) and arginine (R) accelerated the MHC-loading with the HA306-318 peptide in a dose-dependent way (Fig. 1b). No effect was observed with the AR dipeptide lacking the phenolic side chain of the YR dipeptide. The replacement of tyrosine by alanine (A) completely abrogated the effect.

As illustrated in Fig. 1a, the stability of the ligand complex is maintained by an H-bond network formed between conserved residues of the peptide binding site and the backbone of the peptide ligand. A considerable number of these bonds are formed in the immediate vicinity of the P1-pocket. While the arginine residue of the YR dipeptide improves solubility, it may also allow positioning the tyrosine side chain into the P1 pocket by forming a minimal polypeptide backbone. To maximize utilization of the H-Bond network the YR dipeptide was acetylated (Ac-) and amidated (-NH₂) at its N- and C-terminus. Docking of Ac-YR-NH₂ to the P1-pocket of HLA-DR1 suggests that as much as five of the conserved H-bonds can be formed with this minimal peptide (Fig. 2a). Compared to the unmodified YR peptide, which should be able to form H-bonds only with β82N, a more than 10 fold increase in the catalytic activity was observed with Ac-YR-NH₂ (Fig. 2b). A partial effect was observed also with Ac-YR and YR-NH₂, whereas Ac-AR-NH₂ was still completely inactive.

To further confirm the importance of the H-bond network a dipeptide was employed in which tyrosine was replaced by β-homotyrosine (b3hY). The use of this amino acid destroys the typical -N-C-CO- frame by adding an additional C-atom into the tyrosine backbone and results in a total loss of activity when the Ac-b3hYR-NH₂ was used instead of Ac-YR-NH₂ (Fig. 2c). Likewise any replacement of the standard L-amino acids by the respective D-enantiomer resulted in the abrogation of activity (Fig. 2d). This applied both for the Ac-YR-NH₂ as well as for Ac-RY-NH₂. Thus, the stereo-selectivity is so strict that not even the retro-inverse dipeptide composed of D-amino acids show any effect.

Steric requirements, H-bond usage and in particular the failure of dipeptides lacking the phenolic side chain suggested that indeed the P1 pocket was addressed. The b86G pocket has a preference of aromatic and, to a lower extent, also for aliphatic anchor residues. To determine whether these preferences would also be reflected in the catalytic activity of dipeptides a series was tested in which the tyrosine residue of Ac-YR-NH₂ was replaced by one of the two other aromatic amino acids phenylalanine (F) and tryptophane (W) and by the aliphatic amino acids leucine (L), methionine (M), isoleucine (I), and valine (V). In line with the anticipated result strongest increase was observed with the aromatic dipeptides, while the aliphatic ones showed weaker activity. No enhancement was detected with the Ac-ER-NH₂ dipeptide, in which tyrosine was substituted by the non-binding glutamate residue (E).

The mean activity is summarized in Table 1. With soluble HLA-DR1 (DRB1*0101) the highest activity was observed with acetylated/amidated dipeptides containing F, followed by peptides with W and Y. Their catalytic rate enhancement was determined to be 6.08 mM⁻¹, 3.54 mM⁻¹ and 3.39 mM⁻¹, i.e. in the presence of 1 mM of Ac-FR-NH₂ on-rate is amplified by a factor of 7.08. Compared to Ac-FR-NH₂ the dipeptides with aliphatic MLE side chains exhibited only between 12% (L) and 4% (V) of the activity, while substitution with A or E abrogated the effect. Activity was also detected for some unmodified tri- and tetrapeptides. Their activity was within the range of the modified aliphatic dipeptides.

So far, the impact of dipeptides was determined only on the loading of empty MHC molecules. To evaluate their influence on complex dissociation soluble class II MHC molecules were preloaded with the CLIP peptide, which binds to HLA-DR1 with lower affinity than HA306-318. Nevertheless, the stability of the CLIP/HLA-DR1 complex is so high that with 20h less than 10% of the complex decay (Fig. 3b). This changes dramatically, when the dipeptides are added to the incubation. After 5h almost 50% of the MHC molecules have lost the CLIP peptide when Ac-FR-NH₂ is present during the incubation. In line with the catalytic activity determined in antigen-loading assays a weaker dissociation was observed with Ac-YR-NH₂ followed by Ac-LR-NH₂.

Execution of the dissociation experiment in the absence and presence of free high-affinity peptide indicated that it is in fact the ligand-exchange that is catalyzed (Fig. 3c). Despite the presence of dipeptides virtually no complex dissociation is detectable in the absence of free peptide (3c). Only the addition of an excess of unlabelled HA306-318 peptide, which prevents rebinding of released CLIP peptide, allows to generate the typical dissociation curves (3d). Thus, dipeptides with MLE side chains are able not only to mediate antigen-loading but also to induce reversible ligand-exchange.

To formally demonstrate that the MLE side chains of the dipeptides act through the P1 pocket mutants of HLA-DR1 were generated in which the glycine residue β86 at the floor of P1 was replaced by valine (β86V) and by tyrosine (β86Y). While β86V represents the natural dimorphic alternate hindering the access of bulky aromatic residues, the non-natural substitution β86Y has been shown to result in a nearly total blockade of the access to the pocket. However, due to the increased receptiveness of the mutated molecule peptides not using P1 can form the complex even with elevated on-rates.

Since the tyrosine anchor residue HA306-318 requires b86G-pockets for efficient binding, the loading experiments with HLA DR1 mutants were carried out with ABL908-922 (Höpner et al., (2006) J. Biol. Chem. 281 (50), 38535-38542). The peptide is derived from the ABL kinase and does not depend on strong interactions with P1 (Fig. 4a-c, left panels). Experiments with soluble class II MHC revealed that it binds equally strong to wt HLA-DR1 (β86G) and HLA-DR1 (β86G→V) and exhibits the highest spontaneous on-rate with HLA-DR1 (β86G→Y). Analogue to the previous experiments also here the addition of dipeptides resulted in increased loading reactions (Fig. 4a-c, right panels). With wt HLA-DR1 (β86G) the enhancement of ABL908-922 loading corresponded to the result obtained with HA306-318, in which the aromatic dipeptides showed stronger enhancements than the aliphatic Ac-LR-NH₂ peptide. The pattern was reversed when probing the loading of HLA-DR1 (β86G→V). In line with the anchor preferences of the shallow P1 pocket, best enhancement was obtained with Ac-LR-NH₂ while weaker amplification was detected with aromatic dipeptides. No enhancement was observed with HLA-DR1 (β86G-Y). At the highest dipeptide concentration used even a slight reduction was detected, while pCP, an organic MLE used in prior studies, still exhibited an effect. Thus, despite the elevated receptiveness of the HLA-DR1 (β86G→Y), in principle, also with this mutant the on-rate can be further increased by MLE compounds.

To confirm that the rules determined with soluble HLA-DR molecules are also valid with cell surface MHC molecules fibroblast cells were transfected with full length versions of wt HLA-DR1 (β86G) and mutated HLA-DR1 (β86G→V). The loading of these cells with ABL908-922 was determined by FACS and revealed a similar pattern as recorded before with soluble MHC molecules (Fig. 4b). On fibroblasts expressing wt HLA-DR1 strongest enhancement was observed with aromatic dipeptides, while the aliphatic Ac-LR-NH₂ peptide was most effective on HLA-DR1 (β86G→V).

Increased loading of fibroblast cells with peptide antigens also translated directly into improved T cell responses (Fig. 4a). The addition of these cells to a CD4+ T cell hybridoma specific for the ABL epitope (SaABL/G5) induced the strongest response when the loading was carried out in the presence of the dipeptides. The effect is particularly evident at the threshold concentration, in which antigen alone triggers only minute responses. The pattern of enhancement by dipeptides observed here correlates directly to the respective antigen load catalyzed by the dipeptide, so that on wt HLA-DR1 most efficient ABL908-922-specific stimulation was achieved with aromatic dipeptides, on mutated HLA-DR1 (β86G→V) with aliphatic Ac-LR-NH₂.

Lastly, to evaluate the immediate influence on peptide-MLE on the CD4+ T cell response in *in vitro* T cell assays were carried out in the presence of peptide antigens and catalytic peptides. With HA306-318 their influence was tested with the HLA-DR1 restricted mouse T cell hybridoma EvHA/X5 (Fig. 5a and b) and the human T cell line PD2 (Fig. 5 c and d). Titration of the peptide MLE revealed maximal activities at concentrations around 2-3 mM (Fig. 5a and c). At these concentrations the dose response curves for the HA306-318 antigen were shifted up to 50fold towards lower concentrations (Fig. 5b and db). While a half-maximal response in the absence of the dipeptide catalyst was detected at a concentration of 31 ng/ml for EvHA/X5 and of 14 ng/ml for PD2, Ac-FR-NH2 lowered the threshold to 0.65 ng/ml and 0.23 ng/ml, respectively. In line with the data obtained before on soluble HLA-DR1 weaker effects were determined with Ac-LR-NH₂. Thus, short peptides exhibiting MLE-like activity can amplify immune responses in an allele-selective way.

The present date further demonstrate that short peptide fragments can influence the ligand composition of class II MHC molecules. With their catalytic component, the MLE-side chain, they target the polymorphic pocket P1 which triggers ligand-exchange and antigen-loading. The structural requirements of this group are dictated by the steric arrangement of the P1 pocket. MLE-peptides therefore exhibit their effect in an allele-selective way, with the highest activity observed for side chains representing the natural anchor amino acids of the respective P1 pocket.

As shown before for organic MLE compounds the mechanism is based on the stabilization of the peptide-receptive conformation. In class II MHC molecules the pocket P1 seems to play a key-role in this process. First evidence was derived from mutational analysis of HLA-DR1 molecules. Substitutions of residue β86G by tyrosine produced an MHC molecule with elevated receptiveness (Chou CL, Sadegh-Nasseri S. HLA-DM recognizes the flexible conformation of major histocompatibility complex class II. J Exp Med. 2000 Dec 18;192(12):1697-706.). The pocket is located proximal to the binding site of HLA-DM, the chaperone mediating antigen loading inside the MCII compartment. Binding studies suggested that HLA-DM interacts with the flexible hydrophobic P1-pocket and it was speculated that this interaction converts the molecule into a conformation that is highly peptide receptive.

While the active conversion of a non-receptive molecule by HLA-DM has recently been questioned it is undisputed that the chaperone stabilizes the peptide receptive conformation. Experiments lead to a model in which the transition to the non-receptive state is correlated with a loss of pocket P1. Experimental evidence for this hypothesis was taken from the observation that organic compounds such as adamantane ethanol stabilize the receptive state by filing P1. A molecular dynamic (MD) simulation confirmed that the pocket P1 is indeed quickly lost. Calculations based on a crystal structure of the HLA-DR1/HA206-318 complex revealed that after removing the ligand the conformational transitions are largely confined to the β₂-helix (Fig. 7a). The most significant transitions were detected near the P1 pocket, corresponding to shifts of more than 3A. A narrowing of the two helices, albeit to lower extent (1 A), was also observed at the side near the P9 pocket. Most importantly, these shifts translated into a complete loss of the P1 pocket (Fig. 7b). In less than 15 ns the P1 cavity, clearly evident in the cross section of the molecule at the start of the simulation, was filled with side chains or removed by distortions. This collapse was prevented when prior to the MD simulation the Ac-FR-NH₂ was docked into the P1 pocket (supplemental data). The overall shifts recorded during the 15 ns calculation were less that 2.9A (Fig. 7c), and virtually no fluctuations were observed within the P1 pocket.

The MD simulation supports the suggested role of P1 in the transition from receptive to non-receptive class II MHC. Based on this model short peptides stabilize the receptive state by preventing the collapse of P1 with their MLE-like anchor side chain, positioned at the pocket by the backbone interactions with the conserved H-bond network. As loading enhancer 'peptide-MLE' may find application in diagnosis, vaccination and immune therapies. As shown in the amplification of *in vitro* response of CD4+ T cells specific for *influenza virus* or the tumor associated ABL-antigen, the addition of dipeptide derivatives lowers the threshold concentration up to 50fold. Knowledge of the precise binding site may serve as a basis for further optimization by rational design.

### Examples

The following non-limiting examples describe the present invention in more detail. The examples do not intend to limit the invention in any way, they only provide the person skilled in the art with guidance for the use of the dipeptide and methods of the invention. The person skilled in the art will understand that the examples constitute guidance and can be varied on the basis of the different dipeptides to be used.

### Example 1

*Compounds and Reagents.* Designed dipeptides were obtained from EMC microcollection GmbH (Tuebingen, Germany).100mM Stock solutions of dipeptides were made in varying concentration of Me2SO and PBS, however in certain cases instead of PBS, citrate buffer ph 7,4 (150mM citric acid and 300mM disodium hydrogen phosphate), was used to avoid acidic pH. YR and AR were dissolved in phosphate buffered saline (PBS pH 7,4). LRMK, YFR, GYV, Ac-AR-NH2, Ac-ry-NH2, Ac-rY-NH2, Ac-Ry-NH2, Ac-yR-NH2, Ac-Yr-NH2 and Ac- 3 h YR-NH2 were dissolved in 10% Me2SO. Ac-FR-NH2, Ac-YR-NH2, Ac-LR-NH2, Ac-ER-NH2 were dissolved in 15% Me2SO, LPKPPKPV dissolved in 20% Me2SO. Ac-WR-NH2 dissolved in 25% Me2SO and final volume of above dipeptides were made up to 100mM either by PBS or citrate buffer. Ac-VR-NH2, Ac-MR-NH2, Ac-IR-NH2 and Ac-RY-NH2 dissolved in 100% DMSO. IC106-120 (KMRMATPLLMQALPM) (Riberdy, J. M. (1992) Nature 360, 474-477), HA306-318 (PKYVKQNTLKLAT) (Lamb, J. R., (1982) Nature 300, 66-69), human ABL 889 (KGKLSRLKPAPPPPP) were obtained from EMC microcollections GmbH (Tuebingen, Germany).N-terminal biotinylation of above peptides were done. Phycoerythrin- (PE) conjugated streptavidin were purchased from Caltag, and -HLA-DR-PE was obtained from BD Biosciences. Peptide stocks were made in PBS.

*Antibodies and soluble HLA-DR1.* The HLA-DR-specific antibody L243 (ATCC) was purified. Recombinant soluble HLA-DR1 (DRA*0101, DRB1*0101) (Sloan, V. S (1995) Nature 375, 802-806) was produced in S2 insect cells as described (Höpner et al., (2006) J. Biol. Chem. 281 (50), 38535-38542).

*Cells.* Stable transfection of L929 fibroblasts (ATCC) were done to generate L101 (DRB1*0101 b86 G) or L101 (β86V) respectively (Höpner et al., (2006), supra). Epstein-Barr virus-transformed B cell 721.221(DRB1*0101) was obtained from ATCC. EvHA/X5 (DRB1*0101-restricted, HA306-318-specific) were derived from HLA-DR1tg mice (Rosloniec, E. F. (1997) J. Exp. Med. 185, 1113-1122) and were generated after fusion of the CD4+ EvHA T cell line with the BW cell (Höpner et al., (2006), supra). CD4+ T cell line PD2 , HLA-DR1 restricted and HA 306-318 specific (Falk K, et al., (2000) Eur. J. Immunol. 30, 3012-3020), ABL 908-922-specific T cell hybridoma SaABL/G2 was generated after fusing a CD4+ T cell line generated in HLA-DR1 Ag mice with the BW cell.

### Example 2

*Peptide loading*/ *of Soluble HLA-DR Molecules-* For *in vitro* loading experiments 100nM HLA-DR1 was incubated with 40 µg/ml (phosphate-buffered saline, pH 7.4, 37 °C, 1 h). Peptide: MHC complex was detected by ELISA using monoclonal -HLA-DR capture antibody (L243, ATCC) and Eu3-labelled streptavidin (DELFIA,Wallac) (Marin-Esteban, V (2004) J. Biol. Chem. 279,)

### Example 3

*Ligand-exchange of soluble HLA-DR molecules- In vitro* ligand exchange experiments were carried out by loading 1,5 µM of HLA-DR1 with biotinylated IC106-120, for 20 hrs (Höpner et al., (2006), supra) in presence of 5% ethanol. HLA-DR1:IC complex was diluted to 1:15 and incubated with 200µg/ml HA peptide in presence and absence of 10mM dipeptide.

### Example 4

*Peptide Loading of Cell Surface MHC Molecules.* For loading experiments biotinylated antigenic peptides were incubated with 10 x 10⁴ HLA-DR expressing cells/well in presence and absence of dipeptides at 37 °C in Dulbecco's modified Eagle's medium, 5% fetal calf serum in a 96 well V bottom plate for 4 hrs. FACS analysis was done as described previously (Höpner et al., (2006), supra).

### Example 5

*T cell Assays.* For pulse wash experiments 5x 10⁴ HLA-DR-expressing cells/well were loaded for 4 h with antigenic peptides in the presence and absence of dipeptides as described above. Cells were then washed, and 5x104 T cells were added to the culture, which was then incubated for 24 h in Dulbecco's modified Eagle's medium, 5% fetal calf serum in 96-well U-bottom plates. However for permanent assay, HLA-DR expressing cells, T cells and dipeptide stayed together in the reaction for 48 hrs. T cell response was determined in a secondary assay with CTL-L cells (ATCC) as described previously (Falk, K., (2002) J. Biol. Chem. 277, 2709-2715). In experiments with PD-2 cell line, HTR's (as APCs) were pre-irradiated for 520 sec and then used for experiments.

### Example 6

*Docking and Molecular Dynamic Simulations.* In order to get a starting structure, the benzene ring of the phenyl residue and the backbone of the dipeptide were superimposed to the residues Y308 and V309 of the HA-Peptide in the binding groove of the crystal structure 1 DLH. Further, a conformation search with the arginine side chain of the dipeptide was performed to find a optimal orientation of this chain. Subsequently, the complex structure was minimized using the tripos (SYBYL 7.3, Tripos Inc., 1699 South Hanley Rd., St. Louis, Missouri, 63144, USA) and GROMACS (Spoel, D.V.D., et al., J. Comput. Chem., 2005, 26, 1701) force field.
For both, the empty MHC structure and the Ac-FR-NH2 in complex with the MHC, a 15ns molecular dynamic simulation in the GROMACS force field (Spoel, D.V.D., et al., J. Comput. Chem., 2005, 26, 1701) was performed. The simulations were done under physiological conditions. For this purpose the protein and the complex structure were calculated in a 0.9% NaCl solution and 310K. After equilibration over a period of 500ps using a positional restraint of 1000kJmol⁻¹nm⁻² on the backbone of the protein and the ligand molecule, a free simulation was performed for a period of 15ns. During the course of the simulation, the actual frame was stored every 5ps. Viualisation of trajectories and arrangement of the figures were realised using VMD (Humphrey, W., Dalke, A. and Schulten, K., "VMD - Visual Molecular Dynamics", J. Molec. Graphics, 1996, vol. 14, pp. 33-38).

## Claims

1. Method for changing the load state of MHC molecules, comprising the following steps:
a) providing a composition comprising MHC molecules;
d) changing the load state of the MHC molecules by adding at least one dipeptide having the following formula:
XZ
wherein X is an aromatic amino acid and Z is selected from arginine, lysine or histidine, and wherein the dipeptide is modified or unmodified; and
c) isolating the MHC molecules.

2. Method for changing the load state of MHC molecules, comprising the following steps:
a) providing a composition comprising MHC molecules;
b') changing the load state of the MHC molecules by adding at least one dipeptide having the following formula:
XZ
wherein X is an aromatic amino acid and Z is selected from arginine, lysine or histidine, and wherein the dipeptide is modified or unmodified, and by adding at least one ligand of MHC molecules; and
e) isolating the MHC molecules.

3. Method according to claim 1, wherein the MHC molecules according to step (a) are loaded with ligands.

4. Method according to claim 1, wherein the method further comprises a washing step subsequent to step (b).

5. Method according to claim 2, wherein the MHC molecules according to step (a) are loaded with ligands or unloaded.

6. Method according to any one of claims 3 or 4, wherein the change in the load state of the MHC molecules in step (b) means unloading of loaded MHC molecules.

7. Method according to claim 5, wherein the change in the load state of the MHC molecules in step (b') means loading of unloaded MHC molecules with ligands or replacement of ligands of loaded MHC molecules by other ligands.

8. Method according to claim 7, wherein the replacement of ligands of MHC molecules loaded with ligands in step (b') comprises the following steps:
(i) decreasing the load or unloading the MHC molecules loaded with ligands;
(ii) loading the MHC molecules with other ligands.

9. Method according to claim 8, wherein steps (i) and (ii) occur simultanously.

10. Method according to claim 9, wherein the dipeptide is linked to the ligand.

11. Method according to any one of claims 1 to 10, wherein the change in the load state of MHC molecules is carried out at a binding pocket of an MHC molecule, whereby the MHC molecules are MHC class I or II molecules.

12. Method according to claim 11, wherein the binding pocket is a binding pocket of an MHC I molecule.

13. Method according to claim 12, wherein the peptide binding pocket of an MHC I molecule is selected from peptide binding pockets A, B, C, D, E or F.

14. Method according to claim 11, wherein the binding pocket is a peptide binding pocket of an MHC II molecule.

15. Method according to claim 14, wherein the binding pocket of an MHC II molecule is selected from peptide binding pockets P1, P3, P4, P6, P7 and P9.

16. Method according to claim 15, wherein the binding pocket is the binding pocket P1.

17. Method according to any one of claims 1 and 16, wherein the aromatic amino acid is selected from the group consisting of the amino acids phenylalanine, tryptophane and tyrosine.

18. Method according to any one of claims 1 to 17, wherein the dipeptide is modified by at least one compound selected from the group consisting of acetyl, amino, methyl, ethyl, acetyl, nitro and iodo group, polyethylene glycol (PEG) and dioxaoctanoic acid (Doo).

19. Method according to claim 18, wherein the dipeptide is modified by an acetyl group on its N-terminus and/or by an amino group on its C-terminus.

20. Method according to claim 19, wherein the dipeptide is selected from the group consisting of Ac-FR-NH₂, Ac-WR-NH₂ and Ac-YR-NH₂.

21. Method according to any one of claims 1 to 20, wherein the ligands of the MHC molecules from steps (a) and/or (c) are selected from antigens, in particular tumor- or pathogen-specific antigens, tissue-specific self-antigens, antigens of autoreactive cells, peptide antigens and fragments of such peptide antigens, complete proteins, protein mixtures and/or complex protein mixtures.

22. Method according to any one of claims 1 to 21, wherein, in order to modify immune responses, the load of MHC molecules on antigen-presenting cells (APCs) is changed.

23. Method according to claim 22, wherein the immune responses are selected from tumor-specific, pathogen-specific or autoreactive immune responses.

24. Method according to claim 22, wherein the antigen-presenting cells are selected from endogenous or non-endogenous maturated and non-maturated dendritic cells, B-cells or macrophages or other antigen-presenting cells.

25. MHC molecule obtainable by a method according to any one of claims 1 to 24.

26. Use of an MHC molecule according to claim 25 for the preparation of a pharmaceutical composition.

27. Use of an MHC molecule according to claim 25 for the preparation of a pharmaceutical composition for triggering tumor-specific, pathogen-specific or autoreactive immune responses.

28. Use of an MHC molecule according to claim 25 for the preparation of a pharmaceutical composition for the treatment of cancer, infectious diseases or autoimmune diseases.

29. Use according to claim 28, wherein the cancer is selected from colon carcinomas, melanomas, renal carcinomas, lymphomas, acute myeloid leukemia (AML), acute lymphoid leukemia (ALL), chronic myeloid leukemia (CML), chronic lymphocytic leukemia (CLL), gastrointestinal tumors, pulmonary carcinomas, gliomas, thyroid tumors, mammary tumors, prostate tumors, hepatomas, various virus-induced tumors such as, for example, papilloma virus-induced carcinomas (e.g. cervical carcinoma), adenocarcinomas, herpes virus-induced tumors (e.g. Burkitt's lymphoma, EBV-induced B-cell lymphoma), heptatitis B-induced tumors (hepatocell carcinomas), HTLV-1- and HTLV-2-induced lymphomas, acoustic neuroma, cervical cancer, lung cancer, pharyngeal cancer, anal carcinoma, glioblastoma, lymphomas, rectal carcinoma, astrocytoma, brain tumors, stomach cancer, retinoblastoma, basalioma, brain metastases, medulloblastomas, vaginal cancer, pancreatic cancer, testicular cancer, melanoma, thyroidal carcinoma, bladder cancer, Hodgkin's syndrome, meningiomas, Schneeberger disease, bronchial carcinoma, hypophysis tumor, Mycosis fungoides, oesophageal cancer, breast cancer, carcinoids, neurinoma, spinalioma, Burkitt's lymphoma, laryngeal cancer, renal cancer, thymoma, corpus carcinoma, bone cancer, non-Hodgkin's lymphomas, urethral cancer, CUP syndrome, head/neck tumors, oligodendroglioma, vulval cancer, intestinal cancer, colon carcinoma, oesophageal carcinoma, wart involvement, tumors of the small intestine, craniopharyngeomas, ovarian carcinoma, abdomen tumors, ovarian cancer, liver cancer, pancreatic carcinoma, cervical carcinoma, endometrial carcinoma, liver metastases, penile cancer, tongue cancer, gall bladder cancer, leukemia, plasmocytoma, uterine cancer, lid tumor or prostate cancer.

30. Use according to claim 28, wherein the infectious diseases are selected from influenza, malaria, SARS, yellow fever, AIDS, Lyme borreliosis, Leishmaniasis, anthrax, meningitis, viral infectious diseases such as AIDS, Condyloma acuminata, hollow warts, Dengue fever, three-day fever, Ebola virus, cold, early summer meningoencephalitis (FSME), flu, shingles, hepatitis, herpes simplex type I, herpes simplex type II, Herpes zoster, influenza, Japanese encephalitis, Lassa fever, Marburg virus, measles, foot-and-mouth disease, mononucleosis, mumps, Norwalk virus infection, Pfeiffer's glandular fever, smallpox, polio (childhood lameness), pseudo-croup, fifth disease, rabies, warts, West Nile fever, chickenpox, cytomegalic virus (CMV), bacterial infectious diseases such as abort (prostate inflammation), anthrax, appendicitis, borreliosis, botulism, Camphylobacter, Chlamydia trachomatis (inflammation of the urethra, conjunctivitis), cholera, diphtheria, donavanosis, epiglottitis, typhus fever, gas gangrene, gonorrhoea, rabbit fever, Helicobacter pylori, whooping cough, climatic bubo, osteomyelitis, Legionnaire's disease, leprosy, listeriosis, pneumonia, meningitis, bacterial meningitis, anthrax, otitis media, Mycoplasma hominis, neonatal sepsis (Chorioamnionitis), noma, paratyphus, plague, Reiter's syndrome, Rocky Mountain spotted fever, Salmonella paratyphus, Salmonella typhus, scarlet fever, syphilis, tetanus, tripper, tsutsugamushi disease, tuberculosis, typhus, vaginitis (colpitis), soft chancre, infectious diseases caused by parasites, protozoa or fungi, such as amoebiasis, bilharziosis, Chagas disease, Echinococcus, fish tapeworm, fish poisoning (Ciguatera), fox tapeworm, athlete's foot, canine tapeworm, candidosis, yeast fungus spots, scabies, cutaneous Leishmaniosis, lambliasis (giardiasis), lice, malaria, microscopy, onchocercosis (river blindness), fungal diseases, bovine tapeworm, schistosomiasis, sleeping sickness, porcine tapeworm, toxoplasmosis, trichomoniasis, trypanosomiasis (sleeping sickness), visceral Leishmaniosis, nappy dermatitis or miniature tapeworm.

31. Use according to claim 28, wherein the autoimmune disorders are selected from multiple sclerosis (MS), rheumatoid arthritis, diabetes, type I diabetes, systemic lupus erythematosus (SLE), chronic polyarthritis, Basedow's disease, autoimmune forms of chronic hepatitis, Colitis ulcerosa, type I allergic disorders, type II allergic disorders, type III allergic disorders, type IV allergic disorders, fibromyalgia, hair loss, Bechterew's disease, Crohn's disease, Myasthenia gravis, neurodermitis, Polymyalgia rheumatica, progressive systemic sclerosis (PSS), psoriasis, Reiter's syndrome, rheumatic arthritis, psoriasis or vasculitis, or further autoimmune disorders of type I, type II, type III or type IV.

32. Use of an MHC molecule according to claim 25 for the preparation of a pharmaceutical composition for attenuating aggressive immune reactions.

33. Use of at least one dipeptide having the following formula:
XZ
wherein X is an aromatic amino acid and Z is selected from arginine, lysine or histidine, and wherein the dipeptide is modified or unmodified, for the preparation of a pharmaceutical composition.

34. Use of at least one dipeptide according to claim 33 in combination with at least one ligand of MHC molecules for the preparation of a pharmaceutical composition.

35. Use according to claim 34, wherein the ligand is selected from antigens, in particular tumor- or pathogen-specific antigens, tissue-specific self-antigens, peptide antigens and fragments of such peptide antigens, complete proteins, protein mixtures and/or complex protein mixtures.

36. Pharmaceutical composition comprising an MHC molecule according to claim 25, and optionally a pharmaceutically acceptable carrier.

37. Pharmaceutical composition comprising at least one dipeptide having the following formula:
XZ
wherein X is an aromatic amino acid and Z is selected from arginine, lysine or histidine, and wherein the dipeptide is modified or unmodified, and optionally a pharmaceutically acceptable carrier.

38. Pharmaceutical composition comprising at least one dipeptide according to claim 37 in combination with at least one ligand of MHC molecules, and optionally a pharmaceutically acceptable carrier.

39. Pharmaceutical composition according to claim 38 further comprising an MHC molecule according to claim 25.

40. Pharmaceutical composition according to any one of claims 38 or 39, wherein the ligand is selected from antigens, in particular tumor- or pathogen-specific antigens, tissue-specific auto-antigens, peptide antigens and fragments of such peptide antigens, complete proteins, protein mixtures and/or complex protein mixtures.

41. Pharmaceutical composition according to any one of claims 38 to 40, wherein the pharmaceutical composition is a vaccine.

42. Screening method for seeking and identifying new antigens, for detecting specific cytotoxic T-cells or for monitoring a specific T-cell response, comprising the following steps:
a) providing a composition comprising MHC molecules whose load state has been changed by a method according to any one of claims 1 to 24; and
b) determining the interaction of these MHC molecules whose load state has been changed by a method according to any one of claims 1 to 24, with a physiological binding partner of the MHC molecules by means of a biochemical or biophysical detection method.

43. Method according to claim 42, wherein the screening method includes *in vitro* T-cell assays, proliferation assays, ELISPOTS, ELISA methods, chromium-release assays and/or high-throughput screening methods (HTS).


SEQUENCE LISTING

<110>  MAX-DELBRÜCK-ZENTRUM FÜR MOLEKULARE MEDIZIN

<120>  Change of the load state of MHC molecules by dipeptides

<130>  MD01P009EP

<160>  6

<170>  PatentIn version 3.3

<210>  1
<211>  4
<212>  PRT
<213>  Artificial

<220>
<223>  Peptide No. 13 of Table 1

<400>  1

Leu Arg Met Lys
1

<210>  2
<211>  7
<212>  PRT
<213>  Artificial

<220>
<223>  Seq. No. 14 of Table 1

<400>  2

Leu Arg Met Lys Leu Pro Lys
1               5

<210>  3
<211>  8
<212>  PRT
<213>  Artificial

<220>
<223>  See description on page 46.

<400>  3

Leu Pro Lys Pro Pro Lys Pro Val
1               5

<210>  4
<211>  15
<212>  PRT
<213>  Artificial

<220>
<223>  IC106-120 (see description on page 46)

<400>  4

Lys Met Arg Met Ala Thr Pro Leu Leu Met Gln Ala Leu Pro Met
1               5                   10                  15

<210>  5
<211>  13
<212>  PRT
<213>  Artificial

<220>
<223>  HA306-318 (see description on page 46)

<400>  5

Pro Lys Tyr Val Lys Gln Asn Thr Leu Lys Leu Ala Thr
1               5                   10

<210>  6
<211>  15
<212>  PRT
<213>  Artificial

<220>
<223>  human ABL 889 (see description on page 46)

<400>  6

Lys Gly Lys Leu Ser Arg Leu Lys Pro Ala Pro Pro Pro Pro Pro
1               5                   10                  15